(19) Europäisches Patentamt

European Patent Office

Office européen des brevets

(11) **EP 1 865 322 A1**

(12) **EUROPEAN PATENT APPLICATION**
published in accordance with Art. 158(3) EPC

(43) Date of publication:
**12.12.2007 Bulletin 2007/50**

(21) Application number: **06730434.5**

(22) Date of filing: **29.03.2006**

(51) Int Cl.:
*G01N 33/53* (2006.01)    *C12M 1/00* (2006.01)
*C12N 15/09* (2006.01)    *G01N 27/447* (2006.01)
*G01N 37/00* (2006.01)

(86) International application number:
**PCT/JP2006/306486**

(87) International publication number:
**WO 2006/106741 (12.10.2006 Gazette 2006/41)**

(84) Designated Contracting States:
**DE FR GB**

(30) Priority: **31.03.2005 JP 2005100379**

(71) Applicant: **Sony Corporation
Minato-ku
Tokyo 108-0075 (JP)**

(72) Inventor: **OHTO, Yasunori,
SONY-KIHARA RESEARCH CENTER INC.
Tokyo 141-0022 (JP)**

(74) Representative: **Jackson, Jonathan Andrew
D Young & Co
120 Holborn
London EC1N 2DY (GB)**

(54) **BIOREACTION EXECUTION APPARATUS, METHOD OF BIOREACTION EXECUTION, DNA CHIP, INFORMATION PROCESSING UNIT, METHOD OF INFORMATION PROCESSING, PROGRAM AND RECORDING MEDIUM**

(57)    This invention relates to a bioreaction execution system and bioreaction execution method capable of producing electric fields in a flow channel, into which a solution with a target gene contained therein is dropped, on a DNA chip and causing the target gene to electrophoretically migrate, the DNA chip, an information processing system and information processing method, a program, and a recording medium. An AC supply unit 41 supplies an AC current to an electromagnetic induction generator unit 42, the electromagnetic induction generator unit 42 generates electromagnetic induction based on the thus-supplied AC current, and generates a magnetic field in adjacent to a flow channel which is formed on a mounted DNA chip 43 and into which a solution with a target gene contained therein is dropped. One of electric fields produced to negate the thus-generated magnetic field is canceled, while the other electric field is maintained. As the target gene is charged, it is caused to electrophoretically migrate by the electric field, which is maintained in only one direction, in the flow channel formed on the DNA chip 43.

FIG.2

EP 1 865 322 A1

**Description**

Technical Field

This invention relates to a bioreaction execution system and bioreaction execution method, a DNA chip, an information processing system and information processing method, a program, and a recording medium, and specifically to a bioreaction execution system and bioreaction execution method, a DNA chip, an information processing system and information processing method, a program, and a recording medium, all of which have made it possible to improve the accuracy of a detection of a target gene.

Background Art

Practical applications of DNA chips or DNA microarrays (which will hereinafter be collectively called simply "DNA chips" unless they need to be distinguished from each other) have been carried forward in recent years. A DNA chip carries a variety of numerous DNA oligonucleotide strands integrated and immobilized as detection nucleic acids on a substrate surface. By detecting with the DNA chip hybridization between a probe immobilized in a spot on the substrate surface and a target in a sample collected from cells or the like, gene expression in the collected cells can be comprehensively analyzed.
As prior art documents relating to an analysis method, correction method and/or the like of a gene expression abundance obtained by a DNA chip or the like, there are, for example, Patent Document 1 to Patent Document 3.

Patent Document 1: Japanese Patent Laid-open No. 2002-071688
Patent Document 2: JP-A-2002-267668
Patent Document 3: Japanese Patent Laid-open No. 2003-028862

For the provision of an increased opportunity of hybridization between the probe and the target gene, it has been the conventional practice to stir or heat a solution in an expression-analyzing reaction channel into which a sample has been dropped.

Disclosure of Invention

Problems to be Solved by the Invention

To increase the opportunity of hybridization between the probe and the target gene, the frequency of meeting between the probe and the target gene has to be increased. For this purpose, the greater the distance of a movement of the target gene in the expression-analyzing reaction channel, the more preferred. As mentioned above, it has been the conventional practice to stir or heat a solution in an expression-analyzing reaction channel, into which a sample has been dropped, so that the opportunity of hybridization between a probe and a target gene can be increased. With this conventional method, however, it was difficult to control the quantity of energy to be applied to a sample because of the intensity of stirring of the solution or the heat so applied.
On the other hand, intensification of the stirring or raising of the temperature with a view to making greater the movement distance of the target gene and hence increasing the opportunity of hybridization between the probe and the target gene, however, led to an increase in the probability that the intended hybridized state would be destructed.
With the foregoing circumstances in view, the present invention has as an object thereof the provision of an improvement in the detection accuracy of a target gene.

Means for Solving the Problems

A bioreaction execution system according to the present invention is a bioreaction execution system for subjecting a first biological substance, which is immobilized in a reaction region arranged in at least one closed flow channel of a substrate with the flow channel formed thereon, and a second biological substance, which is bioreactive with the first biological substance, to a bioreaction, and is characterized in that it includes an electromagnetic induction generator unit for generating electromagnetic induction to produce an electric field of a predetermined direction along the flow channel such that the second biological substance contained in a solution dropped into the flow channel of the substrate mounted on the system is caused to electrophoretically migrate.
The flow channel formed on the substrate can be circular.
The second biological substance can include a third biological substance specifically bioreactive with the first biological substance and a fourth substance non-specifically bioreactive with the first biological substance, and the electromagnetic induction generator unit can generate electromagnetic induction such that energy to be applied by the electric field of the predetermined direction to cause the electrophoretic migration of the second biological substance in the flow channel can dissociate an association formed by a bioreaction between the first biological substance and the fourth biological substance without dissociating an association formed by a bioreaction between the first biological substance and the third biological substance.

The bioreaction execution system can further include an AC supply unit for supplying an AC voltage to the electromagnetic induction generator unit, the AC voltage to be supplied by the AC supply unit to the electromagnetic induction generator unit can have electric energy sufficient to generate electromagnetic induction such that the energy to be applied by the electric field of the predetermined direction to cause the electrophoretic migration of the second biological substance in the flow channel cannot dissociate the association formed by the bioreaction between the first biological substance and the third biological substance but can dissociate the association formed by the bioreaction between the first biological substance and the fourth biological substance.

The electromagnetic induction generator unit can be provided with a coil-shaped electroconductor for receiving a supply of an alternating current and allowing a current to flow alternately in two directions depending on a polarity of the alternating current, and an electric field canceller for canceling production of one of electric fields, which are produced in two directions to negate a magnetic field generated by the current flowing through the electroconductor, by permitting only a current generated by the one electric field and not permitting a current generated by the other electric field.

The flow channel formed on the substrate can be circular, and the electroconductor and the electric field canceller can be arranged above and below the circular flow channel, respectively, with the circular flow channel located therein.

The substrate can be provided with plural flow channels, which are as defined in claim 5, in a concentric pattern, electroconductors and electric field cancellers, which are as defined in claim 5, can be arranged above and below the flow channels, respectively, with the corresponding flow channels located therein, the electroconductors can each conduct an AC current opposite in polarity to those conducted through adjacent one(s) of the electroconductors, and the electric field cancellers can cancel electric fields of a same direction.

A method according to the present invention for executing a bioreaction is a method for executing the bioreaction in a bioreaction execution system that subjects a first biological substance, which is immobilized in a reaction region arranged in at least one closed flow channel formed on a substrate to permit flowing of a solution dropped onto the substrate, and a second biological substance, which is bioreactive with the first biological substance, to the bioreaction, and is characterized in that it includes generating electromagnetic induction to produce an electric field of a predetermined direction along the flow channel such that the second biological substance contained in the dropped into the flow channel of the substrate is caused to electrophoretically migrate.

In the bioreaction execution system and bioreaction execution method according to the present invention, electromagnetic induction is generated so that an electric field of a predetermined direction is produced along the at least one closed flow channel formed on the substrate. As a result, the second biological substance contained in the solution dropped into the flow channel on the substrate is caused to electrophoretically migrate.

A DNA chip according to the present invention is characterized in that it includes a substrate, a flow channel formed on the substrate and including a concave channel of a closed form, a reaction region arranged in the flow channel to immobilize a first biological substance therein such that the first biological substance is allowed to undergo a bioreaction with a second biological substance contained as a detection target in a solution dropped into the flow channel.

In the DNA chip according to the present invention, the substrate is provided with the flow channel including the concave channel of the closed form, and in the flow channel, there is arranged a reaction region in which a first biological substance bioreactive to the second biological substance as a detection target is immobilized.

An information processing system according to the present invention is an information processing system for executing processing, which determines an energy quantity to be applied for electrophoretic migration of a second biological substance bioreactive with a first biological substance and contained in a solution dropped into at least one closed flow channel formed on a substrate, in a bioreaction execution system for subjecting the first biological substance, which is immobilized in a reaction region arranged in the flow channel, and the second biological substance to a bioreaction, and is characterized in that is includes an acquisition means for acquiring, on the substrate with a solution containing a third biological substance specifically bioreactive with the first biological substance and a fourth substance non-specifically bioreactive with the first biological substance dropped as the second biological substance into the flow channel, parameters corresponding to bioreaction rates of the third biological substance and fourth biological substance in states that different energy quantities have been applied, respectively, a calculation means for calculating, based on the parameters acquired by the acquisition means, the bioreaction rates of the third biological substance and fourth biological substance in the states that the different energy quantities have been applied, and an energy determination means for determining, based on the bioreaction rates calculated by the calculation means, an energy quantity that can dissociate an association formed by a bioreaction between the first biological substance and the fourth biological substance without dissociating an association formed by a bioreaction between the first biological substance and the third biological substance.

An information processing method according to the present invention is an information processing method for an information processing system that executes processing, which determines an energy quantity to be applied for electrophoretic migration of a second biological substance bioreactive with a first biological substance and contained in a solution dropped into at least one closed flow channel formed on a substrate, in a bioreaction execution system for subjecting the first biological substance, which is immobilized in a reaction region arranged in the flow channel, and the second biological substance to a bioreaction, and is characterized in that it includes an acquisition step for acquiring, on the substrate with a solution containing a third biological substance specifically bioreactive with the first biological substance and a fourth substance non-specifically bioreactive with the first biological substance dropped as the second biological substance into the flow channel, parameters corresponding to bioreaction rates of the third biological substance and fourth biological substance in states that different energy quantities have been applied, respectively, a calculation step for calculating, based on the parameters acquired by processing in the acquisition step, the bioreaction rates of the third biological substance and fourth biological substance in the states that the different energy quantities have been applied, and an energy determination step for determining, based on the bioreaction rates calculated by processing in the calculation step, an energy quantity that can dissociate an association formed by a bioreaction between the first biological substance and the fourth biological substance without dissociating an association formed by a bioreaction between the first biological substance and the third biological substance.

A program according to the present invention and a program recorded in a recording medium according to the present invention is a program for making a computer execute processing, which determines an energy quantity to be applied for electrophoretic migration of a second biological substance bioreactive with a first biological substance and contained in a solution dropped into at least one closed flow channel formed on a substrate, in a bioreaction execution system for subjecting the first biological substance, which is immobilized in a reaction region arranged in the flow channel, and the second biological substance to a bioreaction, and make the computer perform processing characterized in that it includes an acquisition control step for controlling, on the substrate with a solution containing a third biological substance specifically bioreactive with the first biological substance and a fourth substance non-specifically bioreactive with the first biological substance dropped as the second biological substance into the flow channel, acquisition of parameters corresponding to bioreaction rates of the third biological substance and fourth biological substance in states that different energy quantities have been applied, respectively, a calculation step for calculating, based on the parameters acquisition of which was controlled by processing in the acquisition step, the bioreaction rates of the third biological substance and fourth biological substance in the states that the different energy quantities have been applied, and an energy determination step for determining, based on the bioreaction rates calculated by processing in the calculation step, an energy quantity that can dissociate an association formed by a bioreaction between the first biological substance and the fourth biological substance without dissociating an association formed by a bioreaction between the first biological substance and the third biological substance.

In the information processing system, information processing method and program according to the present invention, on the substrate with the solution containing the third biological substance specifically bioreactive with the first biological substance and the fourth substance non-specifically bioreactive with the first biological substance dropped as the second biological substance into the flow channel, the parameters corresponding to the bioreaction rates of the third biological substance and fourth biological substance in states that different energy quantities have been applied, respectively, are acquired, the bioreaction rates of the third biological substance and fourth biological substance in the states that the different energy quantities have been applied are calculated based on the parameters, and based on the bioreaction rates, an energy quantity that can dissociate an association formed by a bioreaction between the first biological substance and the fourth biological substance without dissociating an association formed by a bioreaction between the first biological substance and the third biological substance is determined.

Effects of the Invention

According to an aspect of the present invention, a biological substance as a target (for example, a target gene) and a biological substance (for example, a gene employed as a probe) immobilized on a substrate is allowed to associate with each other, and in particular, the biological substance as a target and the biological substance immobilized on the substrate is allowed to effectively associate with each other.

According to another aspect of the present invention, it is possible to provide a DNA chip for the detection of a predetermined biological substance by a bioreaction. Especially when predetermined energy is applied to a biological substance in a solution which is flowing in a flow channel, a biological substance as a target and a biological substance immobilized on a substrate is allowed to effectively associate with each other.

According to a further aspect of the present invention, energy to be applied to a biological substance (for example, a target gene) to be subjected to electrophoretic migration can be determined. In particular, it is possible to determine energy required to increase the frequency of meeting between a biological substance as a target (for example, a target gene) and a biological substance immobilized on a gene and a substrate (for example, a gene used as a probe) and to dissociate a non-specific bioreaction (hybridization).

Brief Description of Drawings

[FIG. 1] A block diagram showing a construction example of an experiment and data-processing system.

[FIG. 2] A block diagram illustrating a construction example of a hybridization unit in FIG. 1.

[FIG. 3] A block diagram depicting a construction example of an AC supply unit in FIG. 2.

[FIG. 4] A diagram for making a description about a DNA chip in FIG. 2.

[FIG. 5] A cross-sectional view for making a description about an electromagnetic induction generator unit in FIG. 2.

[FIG. 6] A diagram for making a description about electric field cancellers.

[FIG. 7] A diagram for making a further explanation about electric field cancellers.

[FIG. 8] A diagram for making a description about a probe and a target gene.

[FIG. 9] A diagram for making a description about energy to be applied upon hybridization.

[FIG. 10] A diagram for making a description about an electric power value to be supplied from an AC supply unit to an electromagnetic induction generator unit.

[FIG. 11] A diagram for making a description about a supply of an alternating current and production of electric fields by magnetic induction.

[FIG. 12] A diagram for making a description about a supply of another alternating current and production of electric fields by magnetic induction.

[FIG. 13] A diagram for making a description about an electrophoretic migration.

[FIG. 14] A diagram for making a description about another electrophoretic migration.

[FIG. 15] A diagram for making a description about another shape example of a DNA chip.

[FIG. 16] A diagram for making explanations about further shape examples of a DNA chip.

[FIG. 17] A block diagram illustrating a construction example of a biological information processing sub-system.

[FIG. 18] A flow chart for making a description about processing in the course of an experiment.

[FIG. 19] A flow chart for making a description about steps of hybridization.

[FIG. 20] A flow chart for making a description about processing steps of preliminary work.

[FIG. 21] A flow chart for making a description about processing for the determination of conditions for supply electric power.

[FIG. 22] A block diagram depicting a construction example of a personal computer.

Description of Reference Symbols

1 experiment and data-processing system, 21 preparation unit, 22 hybridization unit, 23 acquisition unit, 24 expression abundance estimation unit, 25 standardization unit, 26 output unit, 27 memory unit, 28 supply electric energy determination unit, 41 AC supply unit, 42 electromagnetic induction generator unit, 43 DNA chip, 81 expression-analyzing reaction channel, 83 spot, 111 magnetic field generating electroconductors, 112 insulators, 113 electric field cancellers, 122 diode, 141 probe, 142 PM target gene, 143 MM target gene, 161,162 dissociation probability curves, 341,361,381 DAN chips, 401 biological information processing sub-system, 431 supply power value determination unit.

Best Modes for Carrying out the Invention

Meanings of terms employed in this specification will hereinafter be described.

The term "probe" means a biological substance, which is immobilized on a bioassay substrate such as a DNA chip and undergoes a bioreaction with a target.

The term "target" means a biological substance, which undergoes a bioreaction with another biological substance immobilized on a bioassay substrate such as a DNA chip.

The term "biological substance" embraces, in addition to substances formed in vivo such as proteins, nucleic acids and saccharides, genes having base sequences to them and substances derived from them.

The term "bioreaction" means a reaction which two or more biological substances biologically undergo. Its typical example is hybridization.

The term "hybridization" means a complementary-chain (double-strand) forming reaction between nucleic acids having complementary base sequence structures.

With reference to the drawings, a description will hereinafter be made about embodiments of the present invention.

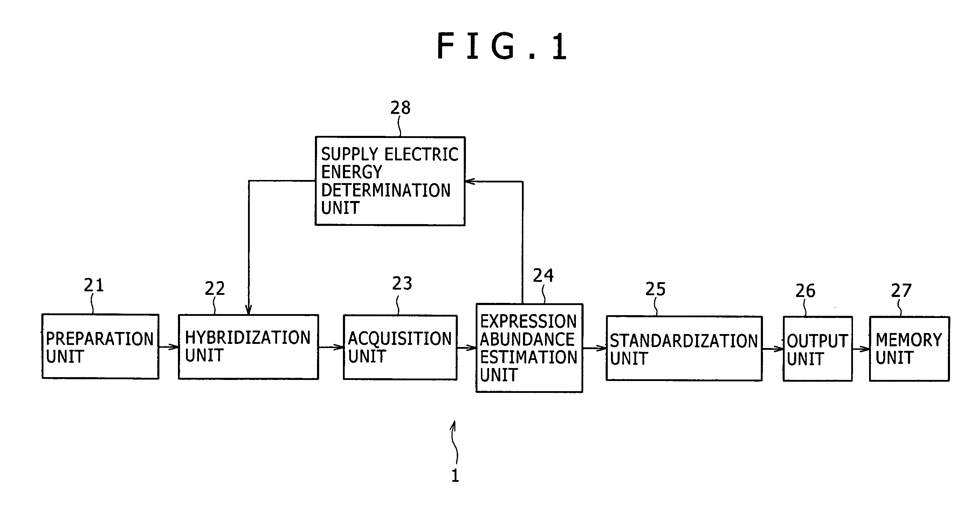

A quantitative measurement of a gene expression abundance is performed by an experiment and data-processing system 1 shown in FIG. 1.

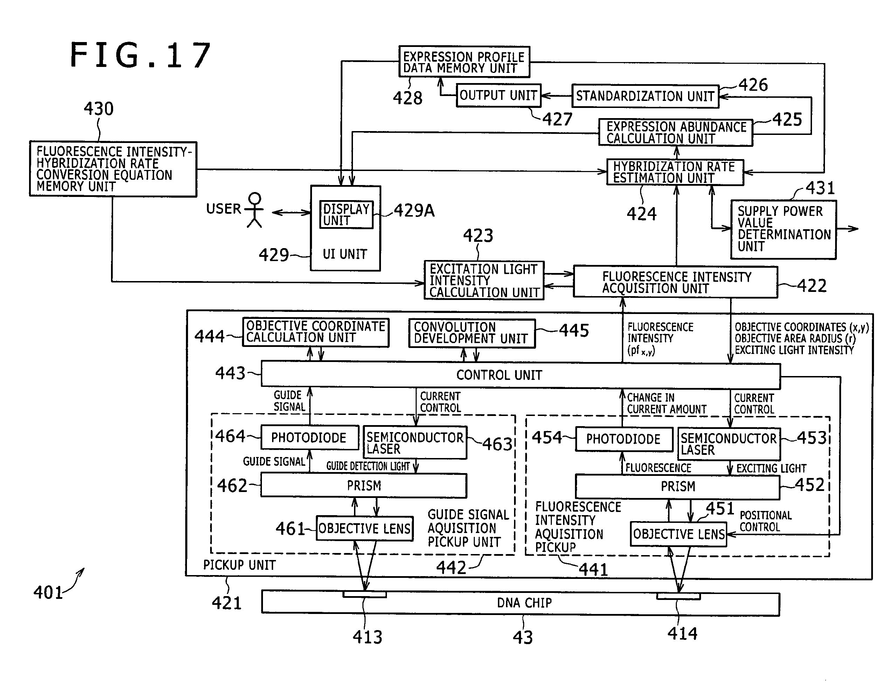

The experiment and data-processing system 1 is constructed of a preparation unit 21, a hybridization unit 22, an acquisition unit 23, an expression abundance estimation unit 24, a standardization unit 25, an output unit 26, a memory unit 27, and a supply electric energy determination unit 28. Among these, the acquisition unit 23, expression abundance estimation unit 24, standardization unit 25, output unit 26, memory unit 27, and supply electric energy determination unit 28 are constructed by a biological information processing sub-system 401 to be described subsequently herein with reference to FIG. 17.

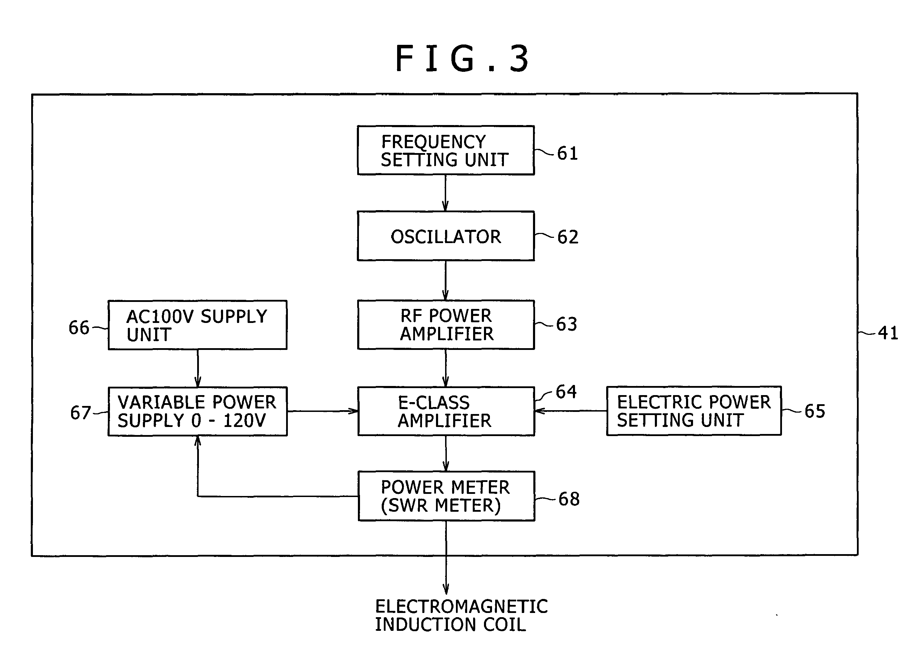

The preparation unit 21 performs a preparation of a target, and also performs a preparation for below-described hybridization making use of a DNA chip. The hybridization unit 22 performs hybridization between a probe and a target. Details of the hybridization unit 22 will be described in detail subsequently herein with reference to FIG. 3. The acquisition unit 23 irradiates a laser beam onto an intercalator bound to a probe and target hybridized together, and as its reflection light, acquires a florescence intensity of the intercalator. The expression abundance estimation unit 24 estimates a hybridization rate on the basis of the acquired fluorescence intensity, and performs estimation processing for an expression abundance of the target gene. The standardization unit 25 performs standardization of data. The output unit 26 outputs expression profile data. The memory unit 27 stores the expression profile data.

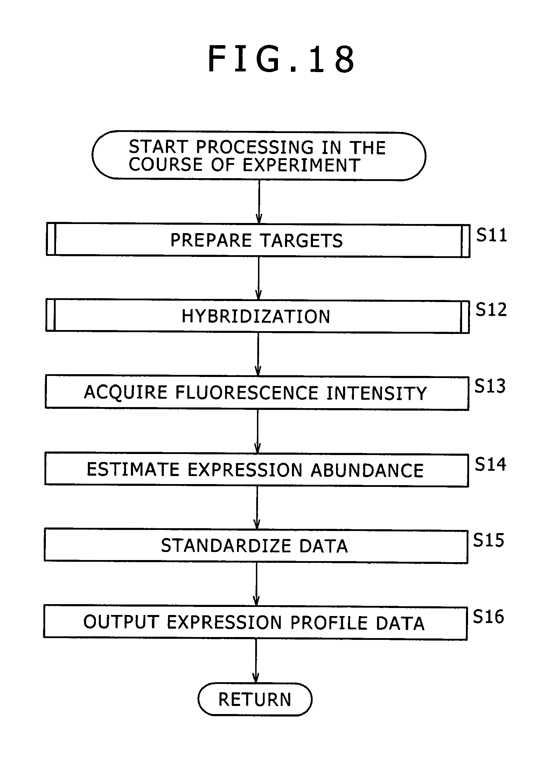

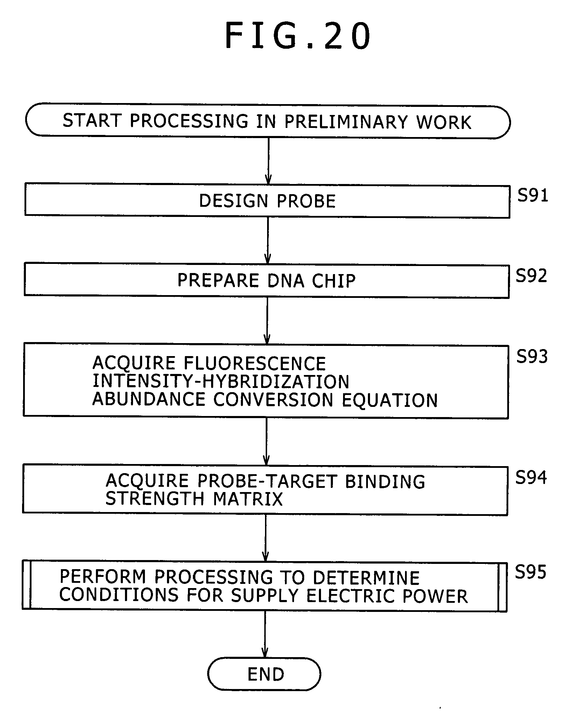

Based on the estimation result of the expression abundance, thex estimation result being supplied by the expression abundance estimation unit 24, the supply electric energy determination unit 28 calculates an electric power value, which the hybridization unit 22 should use in the processing in the course of an experiment to be described subsequently herein with reference to FIG. 18, in the processing of the preliminary work to be described subsequently herein with reference to FIG. 20, and supplies the calculated electric power value to the hybridization unit 22. Using the electric power value so supplied, the hybridization unit 22 executes the processing in the course of the experiment.

About the details of the acquisition unit 23, expression abundance estimation unit 24, standardization unit 25, output unit 26, memory unit 27, and supply electric energy determination unit 28, a description will be made subsequently herein as the biological information processing sub-system 401 with reference to FIG. 17.

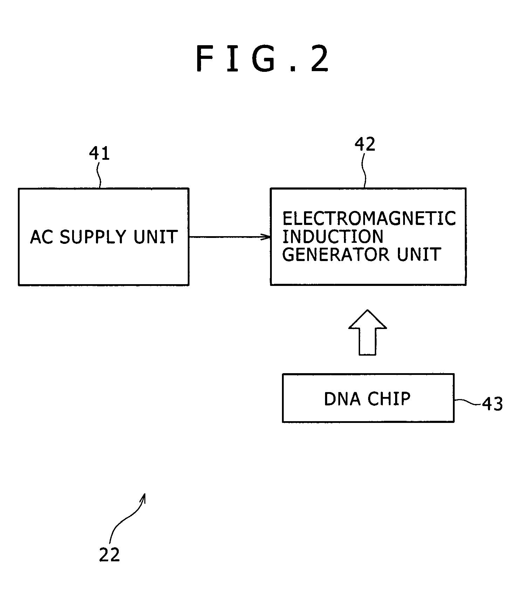

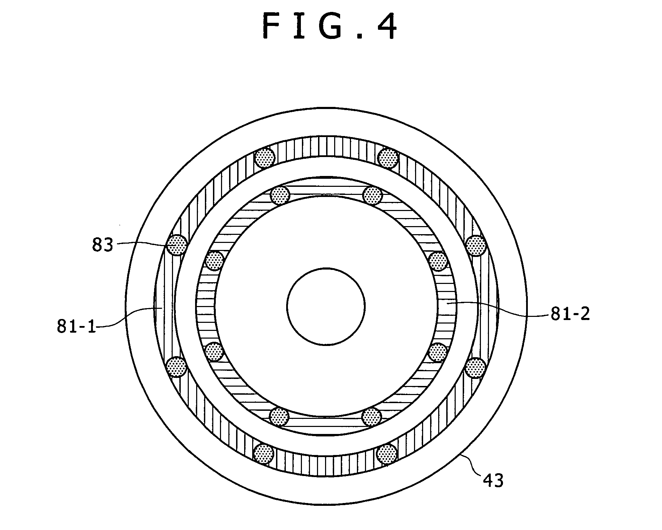

FIG. 2 is a block diagram showing a detailed construction example of the hybridization unit 22 in FIG. 1. The hybridization unit 22 is constructed of an AC supply unit 41 and an electromagnetic induction generator unit 42, and is constructed to permit mounting a DNA chip 43 on the electromagnetic induction unit 42 for hybridization. Arranged on the DNA chip 43 are flow channels in the form of concave channels, into which a solution with the target gene contained therein is dropped. Construction details of the DNA chip 43 will be described subsequently herein with reference to FIG. 4.

The AC supply unit 41 controls the generation of an alternating current adapted to generate electromagnetic induction at the electromagnetic induction generator unit 42, and supplies it to the electromagnetic induction generator unit 42. Details of the AC supply unit 41 will be described subsequently herein with reference to FIG. 3.

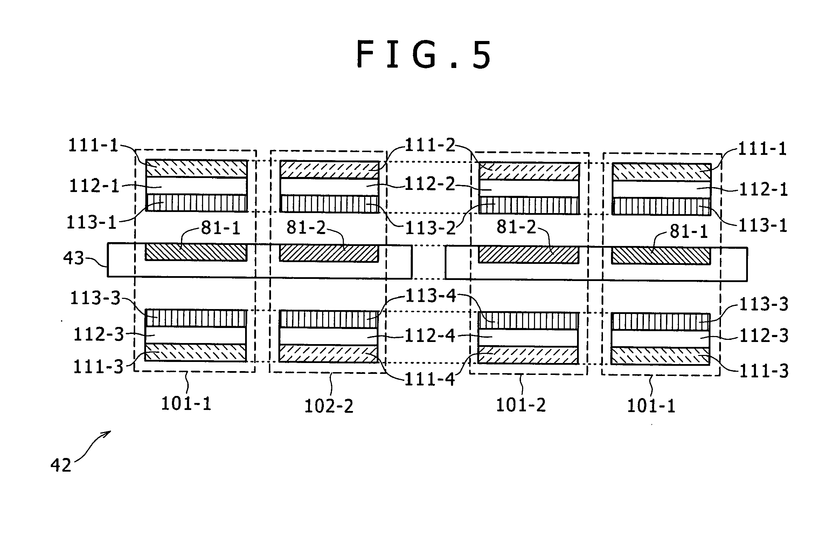

Based on the alternating current supplied from the AC supply unit 41, the electromagnetic induction generator unit 42 generates electromagnetic induction to generates. Adjacent to the flow channels arranged on the thus-mounted DNA chip 43, into which one flow channel the solution with the target gene contained therein is dropped, a magnetic field is, therefore, generated along the one flow channel of the DNA chip 43. In addition, the electromagnetic induction generator unit 42 cancels one of electric fields produced to cancel the above-generated magnetic field, and retains the other electric field. As the target gene is charged, the target gene is caused to electrophoretically migrate in the flow channel formed on the DNA chip 43 under the force of the electric field produced to negate the magnetic field generated by the electromagnetic induction generator unit 42. Details of the electromagnetic induction generator unit 42 will be described subsequently herein with reference to FIG. 5 through FIG. 7.

FIG. 3 is a block diagram illustrating the construction of the AC supply unit 41.

A frequency setting unit 61 sets the frequency of an oscillation by an oscillator 62. The oscillator 62 oscillates a signal of the frequency, which has been set by the frequency setting unit 61, to generate magnetic induction at the electromagnetic induction generator unit 42.

An RF power amplifier 63 amplifies the signal of the predetermined frequency oscillated by the oscillator 62, and supplies the thus-amplified signal to an E-class amplifier 64. Upon receipt of an AC voltage the voltage value of which has been controlled by a variable power supply 67, the E-class amplifier 64 amplifies the AC voltage into an AC voltage having an electric power value set by an electric power setting unit 65 and the frequency of signal supplied from the RF power amplifier 63, and supplies the amplified AC voltage to a power meter (SWR meter) 68.

The electric power setting unit 65 sets an electric power value corresponding to energy applied to subject the target-containing solution to electrophoretic migration on the DNA chip 43 to dissociate only the hybridization of unintended (non-specific) target genes without dissociating the hybridization between the intended (specific) target gene and the probe, and supplies the electric power value to the E-class amplifier 64.

An AC100V supply unit 66 supplies an alternating 100V voltage to the variable power supply 67, for example, by an input through an electric outlet, a predetermined battery or the like. Based on the measurement value of the output voltage of the E-class amplifier 64 as supplied from the power meter 68, the variable power supply 67 transforms the 100V AC voltage, which has been supplied from the AC100V supply unit 66, into a predetermined voltage of from 0 V to 120 V, and supplies the predetermined voltage to the E-class amplifier 64.

The power meter 68 measures an SWR (standing wave ratio, generally also called "matching"), which is a ratio of a maximum to minimum value in a standing wave occurred along an electric wave transmission line. To determine an SWR, a voltage is generally measured, and SWR = $V_{max}$ (maximum value) $/V_{min}$ (minimum value). The power meter 68 supplies the measurement result to the variable power supply 67, and also supplies the supplied AC voltage to the electromagnetic induction generator unit 42 (a magnetic field generating electroconductor 111 to be described subsequently herein, which has the shape of an electromagnetic induction coil).

Referring to FIG. 4, a description will next be made about the DNA chip 43.

On the DNA chip 43, plural closed, circular, concave channels are constructed in the form of concentric circles, and these circular concave channels are used as an expression-analyzing reaction channel 81-1 and an expression-analyzing reaction channel 81-2. In other words, the flow channels formed of the concave channels have neither a starting point nor an ending point, and are continuous. The expression-analyzing reaction channel 81-1 and expression-analyzing reaction channel 81-2 will hereinafter be called simply "the expression-analyzing reaction channel 81" unless they need to be specifically distinguished from each other.

Further, an unillustrated guide for specifying a position in the expression-analyzing reaction channel 81 (a guide 413 in FIG. 17, which will be mentioned subsequently herein) is arranged on the DNA chip 43 at a location suited for specifying the position in the expression-analyzing reaction channel 81.

In the expression-analyzing reaction channel 81, plural spots 83 are formed as reaction regions, and in the respective spots 83, hybridization-verifying probes are immobilized as biological substances (first biological substances) having different gene sequences. Assume that a sample obtained by preparing cells of an organism as an observation target and containing a target gene is dropped into the expression-analyzing reaction channel 81. To the hybridization-verifying probes, targets as biological substances having bases of complementary constructions to the bases of the hybridization-verifying probes (second biological substances) hybridize, respectively. In the spots 83 in the expression-analyzing reaction channel 81, an expression-standardizing control probe may be distributed and arranged at a predetermined plural number of positions as needed. To the expression-standardizing control probe, a target as a biological substance having a base of a complementary construction to the base of the expression-standardizing control probe (second biological substance) hybridizes.

Here, the description has been made about the case that two circular channels of the expression-analyzing reaction channel 81-1 and expression-analyzing reaction channel 81-2 are arranged. Needless to say, the number of circular flow channel(s) of the expression-analyzing reaction channel (s) 81 can also be 1 or any number of 3 or greater.

With reference to FIG. 5 through FIG. 7, a description will next be made about the construction of the electromagnetic induction generator unit 42 in FIG. 2. Firstly, FIG. 5 is a cross-sectional view of the electromagnetic induction generator unit 42 with the DNA chip 43 mounted thereon (cross-sectional view taken along a straight line corresponding to a diameter of the mounted DNA chip 43). Here, the electromagnetic induction generator unit 42 designed to permit mounting the DNA chip 43, which is provided with the two circular channels of the expression-analyzing reaction channel 81-1 and expression-analyzing reaction channel 81-2, will be described as an example.

The electromagnetic induction generator unit 42 is constructed such that in a state with the DNA chip 43 mounted thereon, an electromagnetic inductor 101-1 and electromagnetic inductor 101-2 are located above and below the two circular channels of the expression-analyzing reaction channel 81-1 and expression-analyzing reaction channel 81-2 located therein. Described specifically, in the state that the DNA chip 43 is mounted on the electromagnetic induction generator unit 42, the electromagnetic inductor 101-1 having a similar circular shape as the expression-analyzing reaction channel 81-1 is located above and below the expression-analyzing reaction channel 81-1 held therein, and the electromagnetic inductor 101-2 having a similar circular shape as the expression-analyzing reaction channel 81-2 is located above and below the expression-analyzing reaction channel 81-2 held therein.

The electromagnetic inductor 101-1 is constructed such that on an upper wall of the expression-analyzing reaction channel 81-1, a magnetic field generating electroconductor 111-1, insulator 112-1 and electric field canceller 113-1 are arranged and on a lower wall of the expression-analyzing reaction channel 81-1, a magnetic field generating electroconductor 111-3, insulator 112-3 and electric field canceller 113-3 are arranged. The electromagnetic inductor 101-2 is constructed such that on an upper wall of the expression-analyzing reaction channel 81-2, a magnetic field generating electroconductor 111-2, insulator 112-2 and electric field canceller 113-2 are arranged and on a lower wall of the expression-analyzing reaction channel 81-2, a magnetic field generating electroconductor 111-4, insulator 112-4 and electric field canceller 113-4 are arranged. The magnetic field generating electroconductor 111-1 and magnetic field generating electroconductor 111-3, the insulator 112-1 and insulator 112-3 and the electric field canceller 113-1 and electric field canceller 113-3 have a similar circular shape as the expression-analyzing reaction channel 81-1, while the magnetic field generating electroconductor 111-2 and magnetic field generating electroconductor 111-4, the insulator 112-2 and insulator 112-4 and the electric field canceller 113-2 and electric field canceller 113-4 have a similar circular shape as the expression-analyzing reaction channel 81-2.

Hereinafter, the electromagnetic inductor 101-1 and electromagnetic inductor 102-2 will be called simply "the electromagnetic conductor 101" unless they need to be specifically distinguished from each other, the magnetic field generating electroconductor 111-1 to magnetic field generating electroconductor 111-4 will be called simply "the magnetic field generating electroconductor 111" unless they need to be specifically distinguished from each other, the insulators 112-1 to insulators 112-4 will be called simply "the insulator 112" unless they need to be specifically distinguished from each other, and the electric field canceller 113-1 to electric field canceller 113-4 will be called simply "the electric field canceller 113" unless they need to be specifically distinguished from each other.

The magnetic field generating electroconductor 111 is a coil-shaped electroconductor (electromagnetic induction coil) having a similar circular shape as the expression-analyzing reaction channel 81-1, and upon receipt of an AC voltage generated by the AC supply unit 41, generates a magnetic field. To negate the magnetic field so generated, there is produced a circular magnetic field having a shape conforming with the shape of the circular flow channel of the expression-analyzing reaction channel 81 on the DNA chip 43.

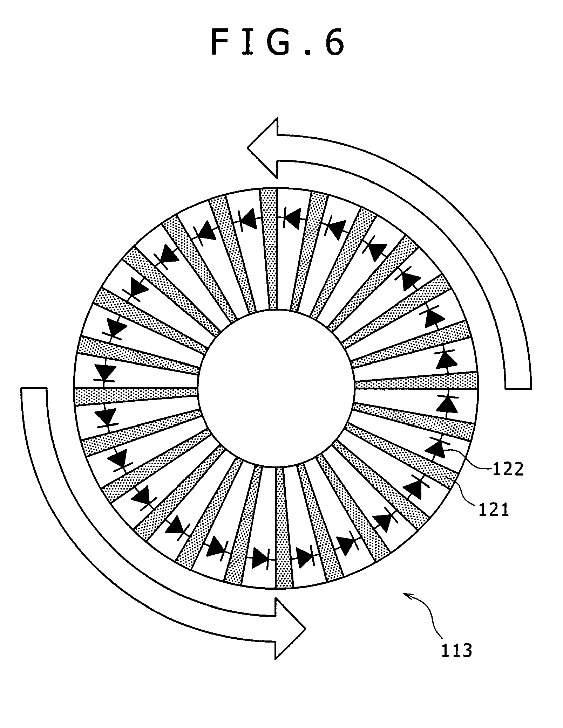

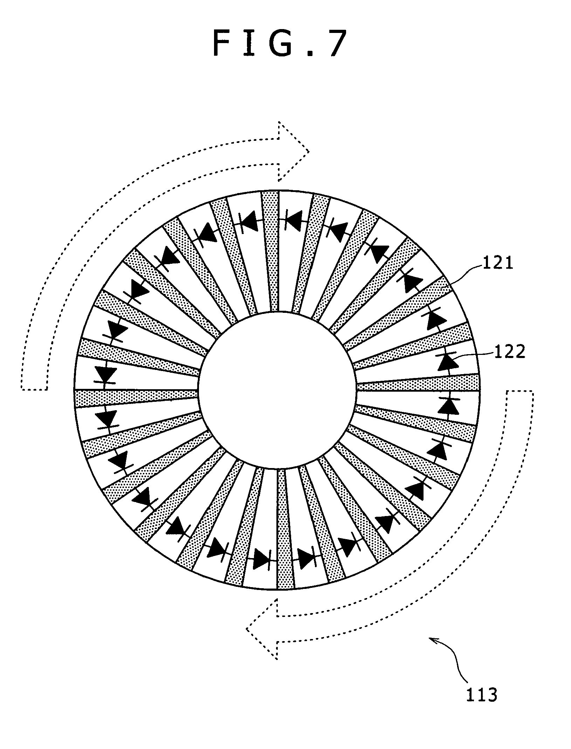

Referring to FIG. 6 and FIG. 7, a description will next be made about the electric field canceller 113. FIG. 6 and FIG. 7 are diagrams illustrating the electric field canceller 113 in a state as observed from a direction perpendicular to a plane of the mounted DNA chip 43.

The electric field canceller 113 is constructed of plural metal portions 121 and plural diodes 122. In FIG. 6 and FIG. 7, the electric field canceller 113 is designed to permit a counterclockwise electric current so that the formation of an electric field can be cancelled.

Referring to FIG. 6, the cancellation of an electric field will be described.

When a magnetic field is formed by electromagnetic induction, for example, perpendicularly to a circular plane of the electric field canceller 113 from the front side toward the rear side in the figure (from the upper side toward the lower side of the paper sheet in FIG. 6), a counterclockwise electric current flows through the electric field canceller 113 to cancel the magnetic field. It is, therefore, possible to cancel the formation of the electric field as a result of the electromagnetic induction.

Referring to FIG. 7, a description will next be made about a case that an electric field is not cancelled, in other words, a case that an electric field is maintained.

When a magnetic field is formed by electromagnetic induction, for example, perpendicularly to the circular plane of the electric field canceller 113 from the rear side toward the front side in the figure (from the lower side toward the upper side of the paper sheet in FIG. 7), a clockwise electric field is formed to cancel the magnetic field. At the electric field canceller 113, however, it is designed to prevent a clockwise electric current from flowing by the diodes 122. Accordingly, the formation of the magnetic field as a result of the electromagnetic induction is maintained.

Described specifically, as a result of a supply of an AC voltage generated by the AC supply unit 41 to the magnetic field generating electroconductor 111, a magnetic field is produced in a direction perpendicular to a flow in the flow channel along the circular flow channel of the expression-analyzing reaction channel 81 of the DNA chip 43 so that a circular electric field of a shape conforming with the shape of the circular flow channel is produced alternately clockwise and counterclockwise. However, only the counterclockwise electric field is cancelled by a counterclockwise electric current generated at the electric field canceller 113. Only an electric field of the clockwise direction is, therefore, produced in the circular channel of the expression-analyzing reaction channel 81 of the DNA chip 43.

In FIG. 6 and FIG. 7, the electric field canceller 113 is designed to permit a counterclockwise electric current so that the formation of an electric field can be cancelled. When the direction of connection of the diodes 122 is opposite, a clockwise electric current is permitted by the electric field canceller 113 so that the formation of an electric field is cancelled. Only a counterclockwise electric is, therefore, produced. At the electric field canceller 113, an electric field in a direction based on the direction of connection of the diodes 122 is, therefore, cancelled to control the direction of an electric field to be produced in the circular channel of the expression-analyzing reaction channel 81.

It is to be noted that circuit (s) in the electric field canceller 113 (electrically-conductive circular paths by the diodes 122) is (are) arranged as many as the expression-analyzing reaction channel(s) 81 to permit electrical fields of different directions to the respective circular channel(s) of the expression-analyzing reaction channels 81 of the DNA chip 43.

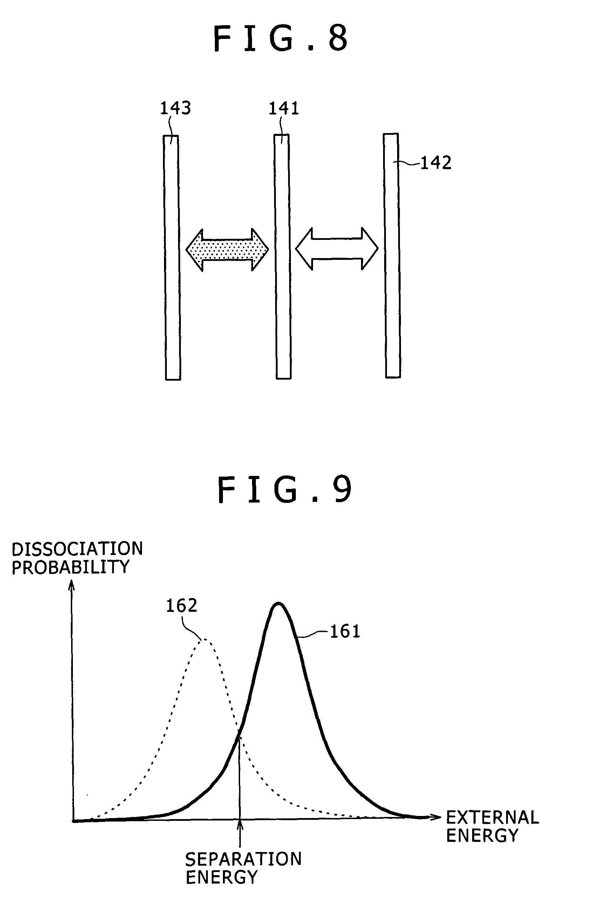

Referring to FIG. 8, a description will next be made about hybridization.

In the circular channel of the expression-analyzing reaction channel 81, a probe 141 arranged in each spots 83 not only hybridizes to a PM (perfect match) target gene 142 as an intended target gene but also may non-specifically hybridize to an unintended MM (mismatch) target gene 143.

Described specifically, the PM target gene 142 is an mRNA as a measurement target, and the MM target 143 is an mRNA the hybridization probability of which with the probe 141 prepared to permit hybridization while assuming the PM target gene 142 is expected to give a largest expression except for the PM target gene 142.

Referring to FIG. 9, a description will be made about energies needed to dissociate the hybridization between the PM target gene 142 and the probe 141 and the hybridization between the MM target gene 143 and the probe 141, respectively.

No matter whether the PM target gene 142 is hybridized with the probe 141 or the unintended MM target gene 143 is hybridized with the probe 141, the hybridization may be dissociated by applying high energy from the outside. Depending on the external energy applied to the hybridized state, the probability that the target and probe dissociate varies. As illustrated in FIG. 9, a dissociation probability curve 161 in the hybridization with the PM target gene 142 and a dissociation probability curve 162 in the hybridization with the MM target gene 143 are shown by plotting the target-probe dissociation probability and the energy applied from the outside along the ordinate and the abscissa, respectively. To dissociate the hybridization with the PM target gene 142, greater energy is required than that needed to dissociate the non-specifically hybridized MM target gene 143.

Assuming that each dissociation probability takes a Gaussian profile centered at a TM (melting temperature) in the probe 141 and the PM target gene 142 or MM target gene 141, the measurement of temperatures at which the hybridization rate becomes 1/4 and 3/4 of that at the TM value makes it possible to estimate such a probability distribution as shown in FIG. 9. The term "Tm value" means the temperature at which 50% of a double-stranded DNA dissociates into single-stranded DNAs. In the respective dissociation probability distributions of the PM target gene 142 and MM target gene 143, an energy point at which a difference between their integrals in a range greater than the energy point becomes maximum can be determined to be separation energy to be applied to the expression-analyzing reaction channel 81 in order to dissociate only the hybridization with the MM target gene 143 without dissociating the hybridization with the PM target gene 142.

The separation energy, which should be applied to the expression-analyzing reaction channel 81 to dissociate only the hybridization with the MM-target gene 143 without dissociating the hybridization with the PM target gene 142, may be set more simply at the average between the TM values of the PM target gene 142 and MM target gene 143.

As the required separation energy is determined as described above, the detection accuracy of a target gene, i.e., the PM target gene 142 can be improved when the force to be applied to the target gene for its electrophoretic migration in the flow channel constructed of the expression-analyzing reaction channel 81 upon hybridization (electrophoretic migration) is controlled to the separation energy which should be applied to the expression-analyzing reaction channel 81 to dissociate the hybridization with the MM target gene 143 without dissociating the hybridization with the PM target gene 142.

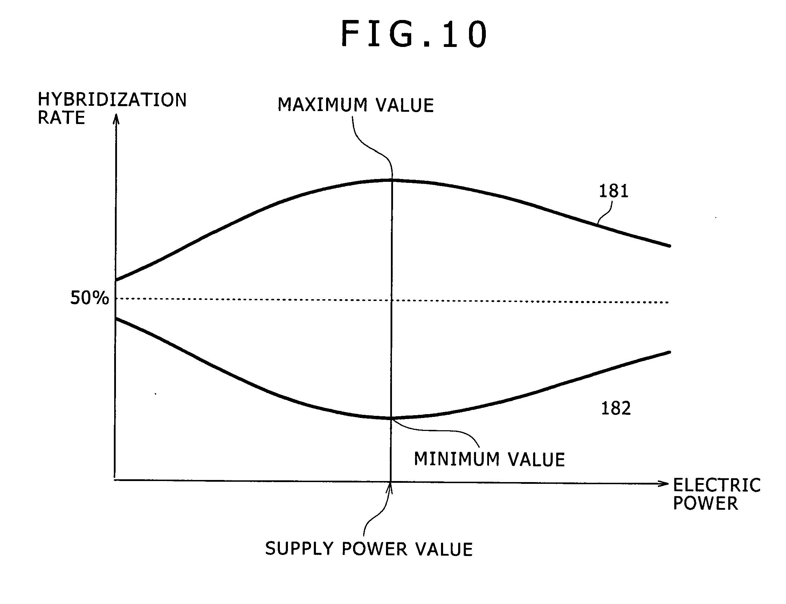

In other words, as illustrated in FIG. 10, when the hybridization rate and the electric energy to be supplied to the magnetic field generating electroconductor 111 are plotted along the ordinate and the abscissa, respectively, an electric power value corresponding to the maximum value of a curve 181, which indicates the hybridization rate between the PM target gene 142 and the probe 141, and the minimum value of a curve 182, which indicates the hybridization rate between the MM target gene 143 and the probe 141, becomes the electric power value to be supplied from the AC supply unit 41 to the electromagnetic induction generator unit 42.

As an alternative, it is also possible to directly conduct the hybridizations between the probes 141 and the PM target gene 142 and MM target gene 143 by an experiment, to express the distributions of their hybridizations with the respective probes 141 by such a binding strength matrix as represented by the following formula (1), and by using the formula (4), then to determine an intensity of electromagnetic induction that maximizes the specificity to the hybridization between the target gene and the probe.

[Mathematical Formula 1]

$$
\begin{pmatrix} p_1 \\ p_2 \\ \vdots \\ p_n \end{pmatrix} = s \times \begin{pmatrix} a_1^1 & a_1^2 & \text{------} & a_1^m \\ a_2^1 & a_2^2 & \text{------} & a_2^m \\ \vdots & \vdots & \diagdown & \vdots \\ a_n^1 & a_n^2 & \text{------} & a_n^m \end{pmatrix} \begin{pmatrix} g_1 \\ g_2 \\ \vdots \\ g_m \end{pmatrix}
$$

$$\cdots\cdots(1)$$

In the formula (1), however, the following formula (2) and formula (3) are held:

[Mathematical Formula 2]

$$s = \left\{ \sum_{i=1}^{n} \sum_{j=1}^{m} a_i^{\ j} \right\}^{-1}$$

$$\cdots (2)$$

[Mathematical Formula 3]

$$1 \leq i \leq m, \ 1 \leq j \leq n, \ 0 \leq p_i, \ a_i^{\ j}, \ g_j$$

$$\cdots (3)$$

In the formula (1), the hybridizations to the respective probes are expressed by the binding strength matrix. Described specifically, fluorescence intensity vectors ($P_1$, $P_2$, $P_3$, ... $P_n$) obtained as a result of binding the intercalator to the spots 83 where the respective probes 141 are arranged can be expressed as a multiplication product of a binding strength matrix expressed by a standardization parameter s, a binding strength matrix $a_1^{\ 1}$ to $a_n^{\ m}$ and gene expression abundance vectors ($g_1$, $g_2$, $g_3$,..$g_m$).

[Mathematical Formula 4]

$$\sum_{i=1}^{n} \sum_{j=1}^{m} \left\{ (a_i^{\ j} - e_i)^2 + (a_i^{\ j} - e^j)^2 \right\}$$

$$\cdots (4)$$

In the formula (4), however, the following formula (5) and formula (6) are held:

[Mathematical Formula 5]

$$e_i = m^{-1} \sum_{j=1}^{m} a_i^{\ j}$$

$$\cdots (5)$$

[Mathematical Formula 6]

$$e^j = n^{-1} \sum_{i=1}^{n} a_i^{\ j}$$

$$\cdots\cdots (6)$$

Described specifically, a generalized inverse matrix of the binding strength matrix of the formula (1), and from the hybridization rates at the probe locations, the respective gene expression abundances can be estimated. The formula (4) is the sum of a variance when the row elements of the binding strength matrix are taken and a variance when its column elements are taken, and the intensity of electromagnetic induction that maximizes the sum is the condition that maximizes the specificity to the hybridization between the PM target gene 142 and the probe 141.

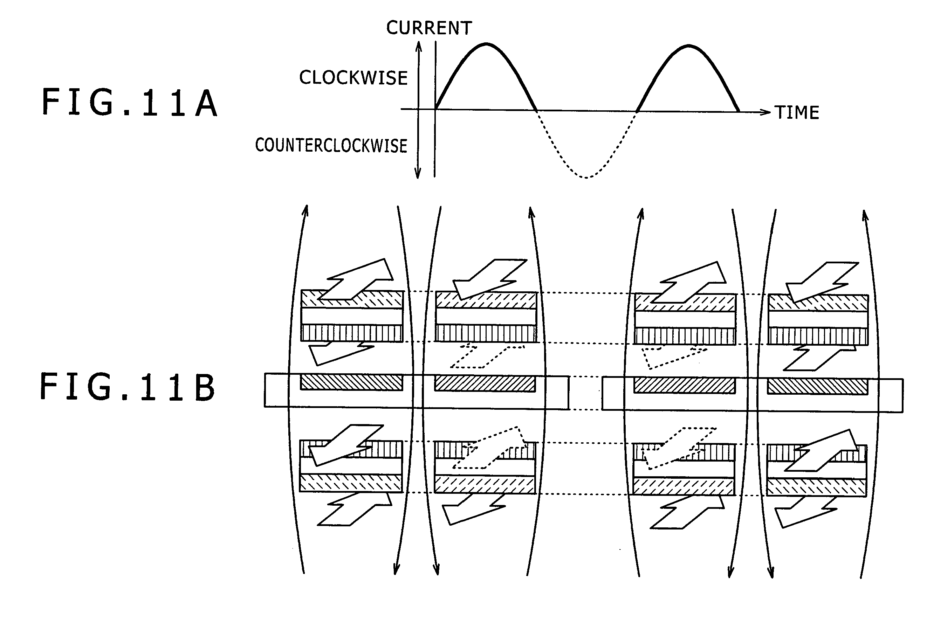

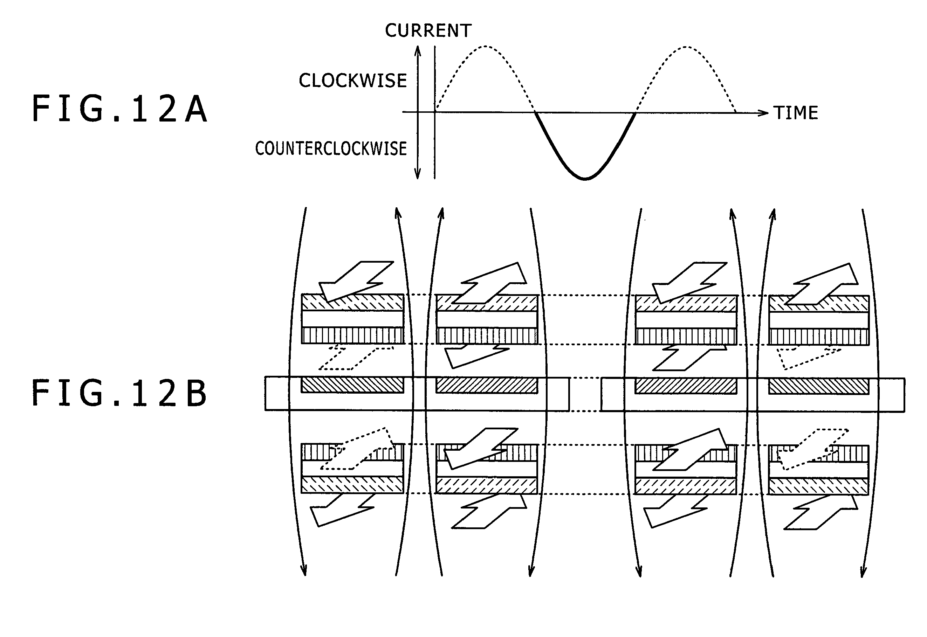

Referring to FIG. 11 and FIG. 12, a description will be made about the supply of an alternating current to the magnetic field generating electroconductor 111 and the production of an electric field by electromagnetic induction.

As shown in a graph A of FIG. 11, clockwise and counterclockwise electric currents alternately flow through each magnetic field generating electroconductor 111 to generate magnetic fields, respectively, because an alternating electric current such as, for example, that shown in the graph A of FIG. 11 is supplied to each magnetic field generating electroconductor 111. It should be understood that as opposed to an alternating electric current supplied from the AC supply unit 41 to the magnetic field generating electroconductor 111-1 and magnetic field generating electroconductor 111-3 in the electromagnetic induction generator unit 42, an alternating electric current of the opposite polarity is supplied to the magnetic field generating electroconductor 111-2 and magnetic field generating electroconductor 111-4 in the electromagnetic induction generator unit 42.

With reference to a diagram B of FIG. 11, a description will firstly be made about a state that alternating electric currents supplied from the AC supply unit 41 to the magnetic field generating electroconductor 111-1 and magnetic field generating electroconductor 111-3 in the electromagnetic induction generator unit 42 are clockwise. To the magnetic field generating electroconductor 111-2 and magnetic field generating electroconductor 111-4 in the electromagnetic induction generator unit 42, on the other hand, alternating electric currents of their opposite polarity (namely, counterclockwise) are supplied. Magnetic fields generated by the magnetic field generating electroconductor 111-1 to magnetic field generating electroconductor 111-4 in directions perpendicular to the flow channels constructed by the expression-analyzing reaction channels 81 are not negated, respectively.

Electric fields are then produced in directions to negate the magnetic fields generated by the magnetic field generating electroconductor 111-1 to magnetic field generating electroconductor 111-4. When counterclockwise electric currents are caused to flow, for example, at the electric field canceller 113-1 to electric field canceller 113-4, the electric fields produced by the magnetic field generating electroconductor 111-1 and magnetic field generating electroconductor 111-3 are cancelled so that only the electric fields produced by the magnetic field generating electroconductor 111-2 and magnetic field generating electroconductor 111-4 are maintained. In the state illustrated in the diagram B of FIG. 11, a clockwise electric field is formed only in the expression-analyzing reaction channel 81-2 to perform an electrophoretic migration of the target gene.

With reference to a diagram B of FIG. 12, a description will next be made about a state that as shown by a solid curve in a graph A of FIG. 12, alternating electric currents supplied from the AC supply unit 41 to the magnetic field generating electroconductor 111-1 and magnetic field generating electroconductor 111-3 in the electromagnetic induction generator unit 42 are counterclockwise. To the magnetic field generating electroconductor 111-2 and magnetic field generating electroconductor 111-4 in the electromagnetic induction generator unit 42, alternating electric currents of a polarity opposite to the alternating electric currents supplied to the magnetic field generating electroconductor 111-1 and magnetic field generating electroconductor 111-2 are also supplied in this state. Magnetic fields in directions perpendicular to the flow channels constructed by the expression-analyzing reaction channels 81 are hence not negated, respectively.

Electric fields are produced in directions to negate the magnetic fields generated as a result of flowing of alternating electric currents to the magnetic field generating electroconductor 111-1 to magnetic field generating electroconductor 111-4. When counterclockwise electric currents are caused to flow, for example, at the electric field canceller 113-1 to electric field canceller 113-4, the counterclockwise electric fields produced by the magnetic field generating electroconductor 111-2 and magnetic field generating electroconductor 111-4 are cancelled so that only the clockwise electric fields produced by the magnetic field generating electroconductor 111-1 and magnetic field generating electroconductor 111-3 are maintained. In the state illustrated in the diagram B of FIG. 12, a clockwise electric field is formed only in the expression-analyzing reaction channel 81-1 to perform an electrophoretic migration of the target gene.

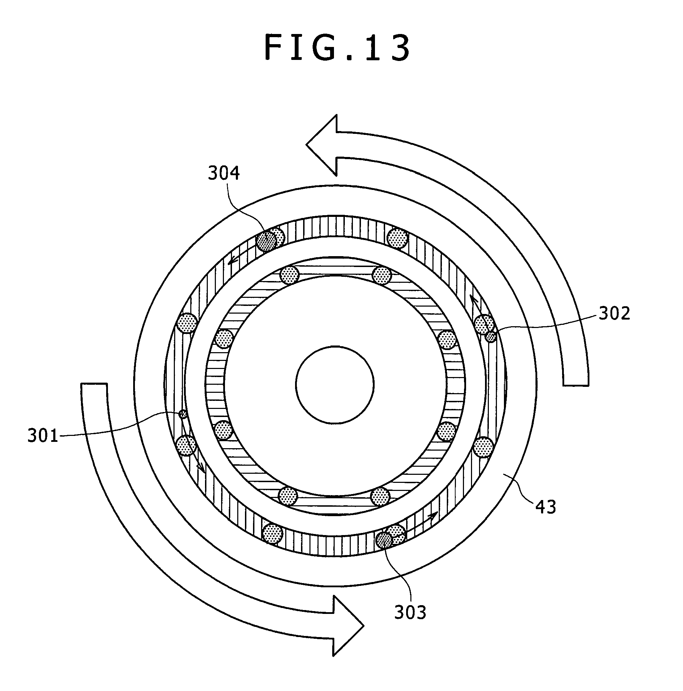

As described with reference to FIG. 11 and FIG. 12, in the expression-analyzing reaction channel 81-1 and expression-analyzing reaction channel 81-2, induced counterclockwise electric fields are cancelled by the electric field cancellers 113, and induced clockwise electric fields are produced in a pulsatory form (a form similar to a half-wave rectification). As the target gene carries a negative charge, it is caused to electrophoretically migrate counterclockwise by an induced clockwise electric field. Because the flow channels of the expression-analyzing reaction channels 81 have no end as shown in FIG. 13, the PM target gene 142 and MM target gene 143 which are caused to electrophoretically migrate under predetermined energy while alternating electric currents are supplied to the magnetic field generating electroconductor 111-1 to magnetic field generating electroconductor 111-4 continue to turn in one direction (in this state, the counterclockwise direction) through the channels, and progressively meet the probes 141 arranged in the flow channels.

When supply electric energy such as that described with reference to FIG. 10 is applied to the electromagnetic induction generator unit 42 such that energy such as that described with reference to FIG. 9 is applied to the electrophoretically-migrating PM target gene 142 and MM target gene 143, the binding between the predetermined probe 141 and the intended PM target gent 142 is not dissociated when they hybridize with each other, but the binding between the predetermined probe 141 and the unintended MM target gene 143 is dissociated by the applied energy when they hybridize with each other. By the application of predetermined energy to target genes 301 to 304 contained in a solution within a flow channel shown in FIG. 13, they successively undergo hybridization with appropriate probes, and after an elapse of sufficient time from the initiation of a supply of an alternating current from the AC supply unit 41 to the magnetic field generating electroconductor 111 in the electromagnetic induction generator unit 42, the state of hybridization becomes very good.

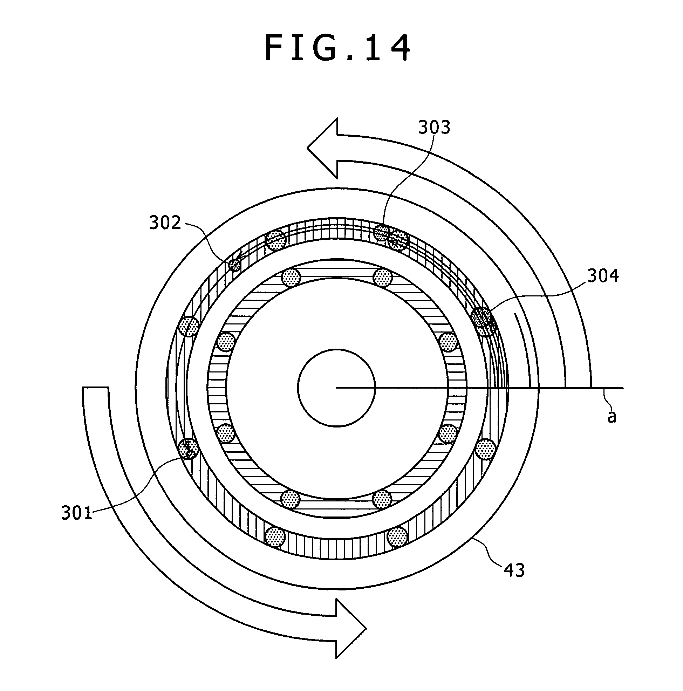

When target genes of different molecular weights are subjected to electrophoretic migration, the target gene the molecular weight of which is low turns quickly, and the turning speed of the target gene becomes slower as its molecular weight becomes greater. Supposing that letter a in FIG. 14 indicates a starting point, the target gene 301 the molecular weight of which is low migrates fast in the flow channel while the target gene 304 the molecular weight of which is high migrates slow in the flow channel. The remaining target genes 302 and 303 also migrate at speeds corresponding to their molecular weights in the flow channel. However, the flow channel of the expression-analyzing reaction channel 81 has no end. The respective target genes 301 to 304, which are electrophoretically migrating under the predetermined energy, continue to turn through the flow channel, and until a sufficient time elapses after the initiation of a supply of an alternating current from the AC supply unit 41 to the magnetic field generating electroconductor 111 in the electromagnetic induction generator unit 42, the target genes 301 to 304 associate with appropriate ones of the probes 141 arranged in the flow channel.

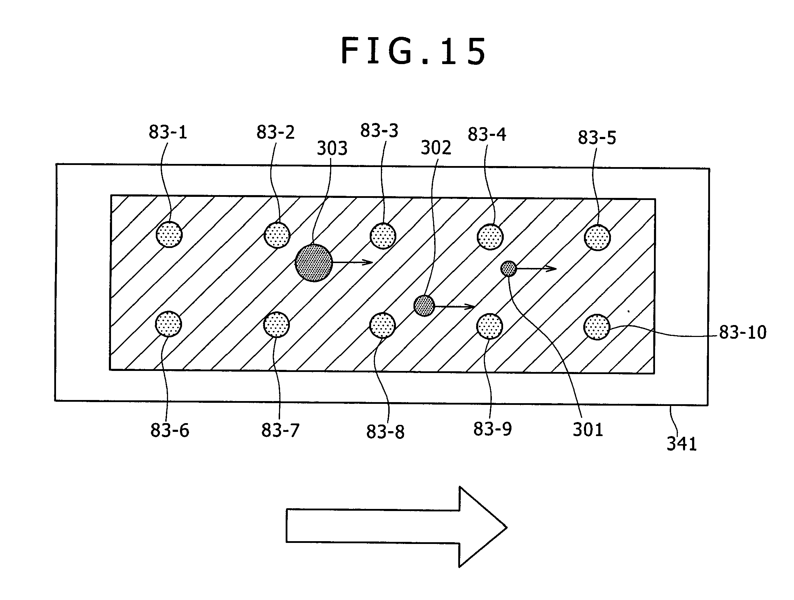

It is to be noted that as in a DNA chip 341 depicted in FIG. 15, each flow channel of a DNA chip may also be, for example, linear other than a circular form. In this case, the flow channel includes a starting point and an ending point, and therefore, target genes 301 to 303 subjected to electrophoretic migration as shown in the figure do not continue to electrophoretically migrate. Compared with the case of a circular flow channel, the association rate between plural probes 141 arranged in spots 83-1 to 83-10 and the corresponding PM target genes is reduced. However, by applying supply power energy such as that described with reference to FIG. 10 to the electromagnetic induction generator unit 42 so that energy such as that described with reference to FIG. 9 can be applied to the electrophoretically-migrating PM target gene 142 and MM target gene 143, only the MM-target gene 143 non-specifically hybridized with the probe 141 can be dissociated without dissociating the PM target gene 142 correctly hybridized with the probe 141.

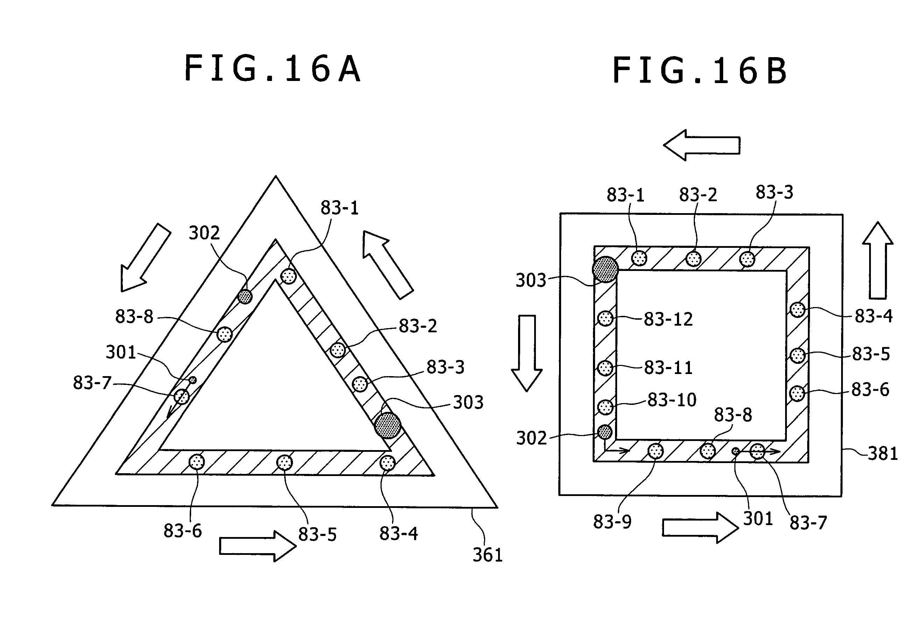

Further, the flow channel constructed by each expression-analyzing reaction channel 81 of a DNA chip is preferably a closed channel even when it has, for example, a form other than a circular form. For example, a DNA chip may have a closed flow channel like a DNA chip 361 shown in a diagram A of FIG. 16 or a closed quadrilateral flow channel line a DNA chip 381 depicted in a diagram B of FIG. 16. Further, the closed flow channel arranged on the DNA chip may also be in a polygonal form other than those mentioned above or may be in a form constructed including a curve. By configuring the flow channel constructed of the expression-analyzing reaction channel 81 into a closed form, electrophoretically-migrating target genes 301 to 303 continue to electrophoretically migrate until they hybridize to intended probes 141. As the MM target gene 143 non-specifically hybridized with the probe 141 is dissociated, the state of hybridization after an elapse of sufficient time since the initiation of a supply of an alternating current from the AC supply unit 41 to the magnetic field generating electroconductor 111 in the electromagnetic induction generator unit 42 becomes very good.

Needless to say, the electromagnetic induction generator unit 42 on which the DNA chip 361 shown in the diagram A of FIG. 16 or the DNA chip 381 depicted in the diagram B of FIG. 16 is constructed to have an electromagnetic inductor 101 in a form conforming with the form of the flow channel of the corresponding DNA chip.

As mentioned above, an intercalator is bound to the promoter and target as biological substances hybridized (bioreacted) on the DNA chip 43. The intercalator emits florescence when exciting light is irradiated.

FIG. 17 shows a construction example of the biological information processing sub-system 401. This biological information processing sub-system 401 is constructed of a pickup unit 421, a fluorescence intensity acquisition unit 422, an exciting light intensity calculation unit 423, a hybridization rate estimation unit 424, an expression abundance calculation unit 425, a standardization unit 426, an output unit 427, an expression profile data memory unit 428, a user interface (UI) unit 429 having a display unit 429A, a fluorescence intensity-hybridization rate conversion equation memory unit 430, and a supply electric energy determination unit 431.

The acquisition unit 23, expression abundance estimation unit 24, standardization unit 25, output unit 26, memory unit 27 and supply electric energy determination unit 28 of the experiment and data-processing system 1 described with reference to FIG. 1 are constructed by the biological information processing sub-system 401 of FIG. 17. Described specifically, the acquisition unit 23 is constructed of the pickup unit 421, fluorescence intensity acquisition unit 422, exciting light intensity calculation unit 423 and fluorescence intensity-hybridization rate conversion equation memory unit 430, the expression abundance estimation unit 24 is composed of the hybridization rate estimation unit 424 and expression abundance calculation unit 425, the standardization unit 25 is composed of the standardization unit 426, the output unit 26 is composed of the output unit 427, the memory unit 27 is composed of the expression profile data memory unit 428, and the supply electric energy determination unit 28 is composed of the supply electric energy determination unit 431.

After completion of hybridization, an intercalator is bound to the promoter 141 and target (PM target gene 142 or MM target gene 143) hybridized (bioreacted) as biological substances on the DNA chip 43 disposed or mounted at a predetermined position. The intercalator emits florescence when exciting light is irradiated.

The pickup unit 421 in FIG. 17 is constructed of a fluorescence intensity acquisition pickup 441, a guide signal acquisition pickup unit 442, a control unit 443, an objective coordinate calculation unit 444, and a convolution development unit 445.

The fluorescence intensity acquisition pickup 441 is a pickup which serves to acquire an image of the expression-analyzing reaction channel 81 of the DNA chip 43. As opposed to the fluorescence intensity acquisition pickup, the guide signal acquisition pickup unit 442 is a pickup for reading the guide 413.

The fluorescence intensity acquisition pickup 441 has an objective lens 451, a prism 452, a semiconductor laser 453 and a photodiode 454. A laser beam (exciting light) emitted by the semiconductor laser 453 enters the objective lens 451 via the prism 452, and the objective lens 451 irradiates the entered laser beam onto the DNA chip 43 (spot 83). The objective lens 451 also causes light, which has been reflected back from the spot 83, to enter the photodiode 454 via the prism 452. In each spot 83, plural probes are immobilized and, when any one of the probes and its corresponding target have hybridized, an intercalator is also bound to both of them. Therefore, the intercalator does not exist between them when the probe and target are not hybridized, but the intercalator exists between them only when they are hybridized with each other. The intercalator emits fluorescence when exciting light is irradiated. The fluorescence condensed by the objective lens 451 is separated from the exciting light by the prism 452, and is caused to enter the photodiode 454.

The greater the hybridization rate, the greater the amount of the intercalator, and accordingly, the higher the intensity of fluorescence emitted from the intercalator. It is, therefore, possible to assay the state of the hybridization (to acquire information on the hybridization) on the basis of the intensity of fluorescence.

The control unit 443 performs control of an electric current to be supplied to the semiconductor laser 453, and controls the intensity of its exciting light. The control unit 443 also reads an output (a change in the amount of the electric current) of the photodiode 454.

The convolution development unit 445 receives from the control unit 443 signals based on changes in the amount of the electric current as outputted from the photodiode 454, and produces pixel-unit image data.

The guide signal acquisition pickup 442 is constructed of an objective lens 461, a prism 462, the semiconductor laser 463 and a photodiode 464. The semiconductor laser 463 emits a laser beam (which functions as guide detection light) on the basis of control from the control unit 443. The prism 462 causes the laser beam from the semiconductor laser 463 to enter the objective lens 461, and the objective lens 461 irradiates this laser beam onto the DNA chip 43. The objective lens 461 receives reflection light from the DNA chip 43, and the prism 462 separates the reflection light from the irradiation laser beam and emits it to the photodiode 464. The photodiode 464 photoelectrically converts the reflection light entered from the prism 462, and outputs it as a guide signal to the control unit 443. The control unit 443 outputs to the objective coordinate calculation unit 444 the guide signal inputted from the photodiode 464. The guide 413 is formed to have a higher (or lower) reflectivity compared with the other areas of the DNA chip 43. Based on the levels of guide signals supplied from the guide signal acquisition pickup 442 via the control unit 443, the objective coordinate calculation unit 444 calculates the position of the guide 413 and the position (coordinates) of the guide signal acquisition pickup 442.

Based on the position of the guide signal acquisition pickup 442 as calculated by the objective coordinate calculation unit 444, the control unit 443 controls the position of the fluorescence intensity acquisition pickup 441 (the objective lens 451). The guide signal acquisition pickup 442 and fluorescence intensity acquisition pickup 441 are fixed relative to each other in a predetermined positional relation.

Upon receipt of an input of fluorescence intensities ($pf_{x,y}$) from the respective spots 83 (their coordinates (x,y)) as outputted from the photodiode 454 of the fluorescence intensity acquisition pickup 441, the fluorescence intensity acquisition unit 422 outputs data on the fluorescence intensities to the hybridization rate estimation unit 424. To the control unit 443, the fluorescence intensity acquisition unit 422 also outputs control signals that control the objective coordinates (x,y) and objective area radius (r) of the objective lens 451 of the fluorescence intensity acquisition pickup 441 over the DNA chip 43 and the intensity of exciting light. The control unit 443 controls the objective lens 451 on the basis of the control signals. As a consequence, the objective lens 451 is arranged at the predetermined coordinates (x, y) over the DNA chip 43, the radius (objective area radius) (r) of irradiation area of the laser beam emitted from the objective lens 451 is controlled at a predetermined value, and the intensity of the laser beam (the intensity of exciting light) is controlled to a predetermined value.

To the exciting light intensity calculation unit 423, the fluorescence intensity acquisition unit 422 outputs a fluorescence intensity supplied from the control unit 443. Based on a conversion equation stored in the fluorescence intensity-hybridization conversion formula memory unit 430 and other necessary parameters, the fluorescence light intensity calculation unit 423 calculates an optimal exciting-light intensity and outputs the thus-calculated and obtained exciting-light intensity to the fluorescence intensity acquisition unit 422. Based on the exciting-light intensity from the fluorescence light intensity calculation unit 423, the fluorescence intensity acquisition unit 422 controls an electric current to be supplied to the semiconductor laser 453 and causes exciting light of a predetermined intensity to be emitted from the semiconductor laser 453.

Upon receipt of image data based on fluorescence intensities as supplied from the fluorescence intensity acquisition unit 422 or image information such as expression profile data stored beforehand in the expression profile data memory unit 428, the hybridization rate estimation unit 424 performs processing to estimate the intensity of the exciting light as needed. In addition, the hybridization rate estimation unit 424 prepares a conversion equation to unambiguously determine a hybridization rate from a fluorescence intensity, and processes the supplied image data.

Moreover, the hybridization rate estimation unit 424 calculates a hybridization rate on the basis of the thus-processed image data. In preliminary work to be described subsequently herein with reference to FIG. 20, a hybridization rate calculation unit 85 calculates the hybridization rates of the PM target gene 142 and MM target gene 143 in the DNA chip 43 for the preliminary work and supplies them to the supply power value determination unit 431.

The user interface unit 429 displays, on the display unit 429A, an image corresponding to the processed image data inputted from the hybridization rate estimation unit 424.

The expression abundance calculation unit 425 estimates an expression abundance, which corresponds to an fluorescence intensity, by determining the binding strength of the target to the probe on the basis of an output from the hybridization rate estimation unit 424. The standardization unit 426 performs standardization processing while making use of an expression-standardizing control probe. The output unit 427 supplies the standardized data to the expression profile data memory unit 428. The expression profile data memory unit 428 stores, as expression profile data, the data supplied from the output unit 427. The data stored in the expression profile data memory unit 428 are supplied to the user interface unit 429 and displayed on the display unit 429A, as needed. The data outputted from the expression abundance calculation unit 425 are also displayed on the display unit 429A as needed.

The fluorescence intensity-hybridization rate conversion equation memory unit 430 stores beforehand a conversion equation which unambiguously determines the relationship between each fluorescence intensity and its corresponding hybridization rate (which may be in the form of data for conversion instead of necessarily developing such an equation).

Based on the hybridization rates of the PM target gene and MM target gene 143 in the DNA chip 43 for the preliminary work as calculated by the hybridization rate calculation unit 85 in the preliminary work to be described subsequently herein with reference to FIG. 20, the supply power value determination unit 431 determines the supply power value of an alternating voltage, which the AC supply unit 41 should generate and supply to the electromagnetic induction generator unit 42 in hybridization to be performed in the processing in the course of an experiment to be described subsequently herein with reference to FIG. 18, and supplies it to the AC supply unit 41 in the hybridization unit 41.

The processing in the course of an experiment to be performed by the experiment and data-processing system 1 of FIG. 3 will next be described with reference to a flow chart of FIG. 18.

In step S11, the preparation unit 21 firstly prepares targets. Described specifically, a sample with cells contained therein is collected, and processing is performed to denature and eliminate proteins from the sample. By extraction and fragmentation of RNAs (ribonucleic acids) and extraction and fragmentation of DNAs (deoxyribonucleic acids), targets including the PM target gene 412, the MM target gene 413 and the like are prepared.

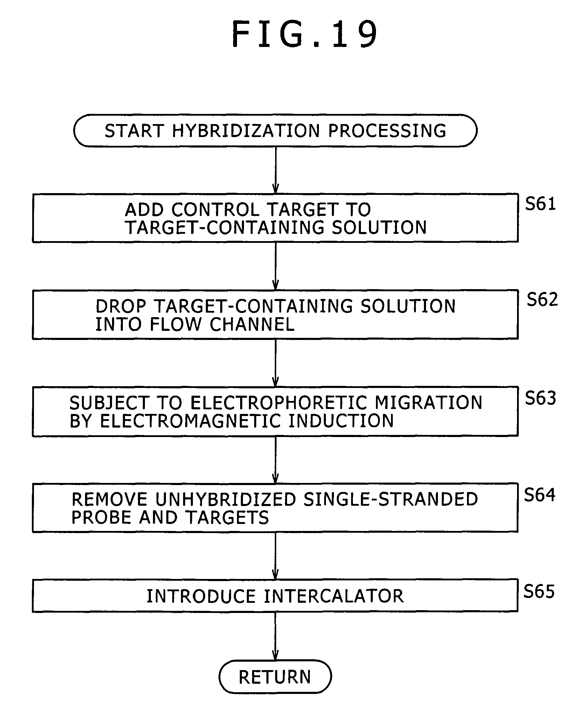

In step S12, hybridization to be described subsequently herein with reference to FIG. 19 is performed.

In step S13, the acquisition unit 23 acquires a fluorescence intensity. Described specifically, the fluorescence intensity acquisition unit 422 drives the fluorescence intensity acquisition pickup 441 via the control unit 443, and makes the semiconductor laser 453 emit a laser beam as exciting light. This exciting light enters the objective lens 451 via the prism 452, and the objective lens 451 irradiates this exciting light onto the expression-analyzing reaction channel 81 on the DNA chip 43.

As mentioned above, an intercalator is bound to the probe 141 and target (the PM target gene 142 or the MM target gene 143) hybridized (bioreacted) as biological substances, and upon receipt of exciting light irradiated from the fluorescence intensity acquisition pickup 441, produces fluorescence. This fluorescence is condensed by the objective lens 451, and is caused to enter the photodiode 454 via the prism 452. Corresponding to the fluorescence so entered, the photodiode 454 outputs an electric current. The control unit 443 makes the convolution development unit 445 convert a signal, which corresponds to the electric current, into an image signal, and outputs a signal, which corresponds to a fluorescence intensity obtained by the conversion, to the fluorescence intensity acquisition unit 422.

The control unit 443 changes the position of the objective lens 451 on the basis of the position of the guide 413. At this time, a laser beam, which the semiconductor laser 463 of the guide signal acquisition pickup 442 has emitted as guide detection light, enters the objective lens 461 via the prism 462, an the objective lens 461 irradiates the guide detection light onto the DNA chip 43. The intensity of reflection light from the guide detection light becomes higher (or weaker) when the guide detection light is irradiated onto the guide 413. This reflection light enters the prism 462 via the objective lens 461, and enters the photodiode 464 from the prism 462. The objective coordinate calculation unit 444 acquires a guide signal from the photodiode 464 via the control unit 443, and based on the signal, calculates the coordinates of the guide signal acquisition pickup 442 (thus, the fluorescence intensity acquisition pickup 441 integrated with the guide signal acquisition pickup) to determine at which position of the guide 413 of the DNA chip 43 is located. Based on the coordinates, the control unit 443 causes the guide signal acquisition pickup 442 (the fluorescence intensity acquisition pickup 441) to move at a constant speed (to perform scanning).

As described above, the fluorescence intensity acquisition pickup 441 is moved to scan the expression-analyzing reaction channel 81 at a predetermined speed, and image signals at the respective coordinates are outputted from the fluorescence intensity acquisition pickup 441.

In step S14, the expression abundance estimation unit 24 performs expression abundance estimation processing on the basis of the fluorescence intensities so acquired. By this processing, the calculations of a hybridization rate and reliability are performed to calculate the expression abundance of the gene.

In other words, the image data based on the fluorescence intensity as supplied from the fluorescence intensity acquisition unit 422 is processed by the hybridization rate estimation unit 424 to estimate the hybridization rate. By the expression abundance calculation unit 425, the gene expression abundance corresponding to the florescence intensity is estimated based on the binding strength of the target to the probe.

In step S15, the standardization unit 25 (standardization unit 426) then performs data standardization processing to correct variations in hybridization depending on the positions of the spots 83 in the expression-analyzing reaction channel 81. This standardization includes, for example standardization by expression-standardizing control probes. Described specifically, based on fluorescence values corresponding to hybridization rates at expression-standardizing control probes distributed and arranged at predetermined plural positions in the expression-analyzing reaction channel 81 (in the solution employed in the experiment, control targets have been added beforehand as targets for the expression-standardizing control probes in step S61 of FIG. 19 to be described subsequently herein), correction values are determined. By dividing the fluorescence values at the respective pixels with fluorescence values obtained by the correction values, normalization is performed. By this normalization, the variations in hybridization depending on the positions of the spots 83 in the expression-analyzing chemical channel 81 are corrected.

In step S16, the output unit 26 (output unit 427) outputs expression profile data, and the process is ended. Described specifically, the image data obtained as described above are supplied from the output unit 26 (output unit 427) to the memory unit 27 (expression profile data memory unit 428) and are recorded there.

By the processing as described above, the expressions of genes contained in the sample can be comprehensively analyzed. If the hybridization at this time is in such a state that the MM target gene 143 is not hybridized with the probe 141 but the PM target gene 142 is hybridized with the probe 141 as firmly as possible, the detection accuracy of the target gene is improved.

With reference to a flow chart of FIG. 19, a description will next be made about hybridization performed in step S12 of FIG. 18.

In step S61, the preparation unit 21 adds a control target, which is a target to the expression-standardizing control probe, to the solution prepared by the processing in step S11 and containing the PM target gene 412 and the like.

In step S62, the preparation unit 21 drops the solution, in which the target and control target are contained, into the flow channel of the expression-analyzing reaction channel 81 of the DNA chip 43 to be mounted in the hybridization unit 22, and the DNA chip 43 is mounted in the electromagnetic induction generator unit 42 of the hybridization unit 22.

In step S63, the hybridization unit 22 causes the PM target gene 142 and MM target gene 143, which are contained in the solution dropped into the expression-analyzing reaction channel 81 of the DNA chip 43, to electrophoretically migrate in a predetermined direction.

Described specifically, the hybridization unit 22 supplies an alternating current from the AC supply unit 41 to the electromagnetic induction generator unit 42. As described above by using the formula (1) to formula (6), the alternating current has a power value corresponding to separation energy to be applied to the expression-analyzing reaction channel 81 to dissociate only the hybridization between the probe 141 and the MM target gene 143 without dissociating the hybridization between the probe 141 and the PM target gene 142. As described with reference to FIG. 5 through FIG. 7, an electric field is formed by electromagnetic induction in the predetermined direction in the flow channel of the expression-analyzing reaction channel 81 of the DNA chip 43 so that the PM target gene 142 and MM target gene 143 in the solution dropped into the expression-analyzing reaction channel 81 of the DNA chip 43 are caused to electrophoretically migrate in the predetermined direction.

A flow channel is not provided with any ending point, for example, in the expression-analyzing reaction channel 81 that forms the closed circular flow channel described with reference to FIG. 4 or in the expression-analyzing reaction channel 81 that forms such a closed polygonal flow channel as described with reference to FIG. 16. Therefore, the electrophoretically-migrating PM target gene 142 continues to migrate until it hybridizes to the intended probe 141, and the MM target gene 143 non-specifically hybridized with the probe 141 is dissociated by energy applied thereto. The state of hybridization after an elapse of sufficient time since the initiation of a supply of an alternating current from the AC supply unit 41 to the magnetic field generating electroconductor 111 in the electromagnetic induction generator unit 42, therefore, becomes very good.

In step S64, the hybridization unit 22 removes probes not immobilized by hybridization, specifically, the single-stranded probe and target from the DNA chip 43 by washing after an elapse of sufficient time from the initiation of a supply of an alternating current from the AC supply unit 41 to the magnetic field generating electroconductor 111 of the electromagnetic induction generator unit 42.

In step S65, the hybridization unit 22 introduces the intercalator between the double-strand bound probe 141 and target, and the processing returns of step S12 of FIG. 18 and advances to step S13.

Incidentally, in step S63, as described with reference to FIG. 13 and FIG. 14, the PM target gene 142 and MM target gene 143 in the flow channel of the expression-analyzing reaction channel 81 of the DNA chip 43 are provided with the predetermined energy by electromagnetic induction and are caused to electrophoretically migrate. Even when the MM target gene 143 is once hybridized to the probe 141, the non-specific hybridization has high possibility of being dissociated because energy sufficient to cause dissociation is applied. Further, the PM target gene 142 is provided with a higher opportunity of binding to the probe 141, and therefore, the hybridization rate is improved.

By the processing as described above, the state of hybridization after an elapse of sufficient time since the initiation of a supply of an alternating current from the AC supply unit 41 to the magnetic field generating electroconductor 111 of the electromagnetic induction generator unit 42 becomes very good. The detection accuracy of the target gene is hence improved.

It is to be noted that the DNA chip 43 employed in the process in the course of the above-mentioned experiment and the solution dropped into the flow channel of the expression-analyzing reaction channel 81 are provided by the preliminary work. Further, the supply power value of the optimal AC voltage, which is generated by the AC supply unit 41 and is supplied to the electromagnetic induction generator unit 42 in the hybridization performed in the processing in the course of the experiment described with reference to FIG. 18, is also determined in the preliminary work.

Referring to a flow chart of FIG. 20, a description will be made about the processing in the preliminary work.

Firstly, the designing of the probe 141 to be arranged on the DNA chip 43 is conducted in step S91.

In step S92, the preparation of the DNA chip 43 is next carried out by immobilizing the designed probe 141 in spots 83 in the flow channel of the expression-analyzing reaction channel 81 of the DNA chip 43.

In step S93, calibration is performed to acquire a fluorescence intensity-hybridization rate conversion equation. Described specifically, various parameters required for the processing in the course of the experiment are acquired by using target solutions with the PM target 142 contained in predetermined amounts and performing the acquisition, processing and/or the like of hybridization and fluorescence intensities.

In step S94, the acquisition of the probe-target binding strength matrix for the estimation of an expression abundance as expressed by the above-described formula (1) to formula (3) is performed.

It is to be noted that the processing in step S93 and step S94 are performed, for example, by using the biological information processing sub-system 401 described with reference to FIG. 17.

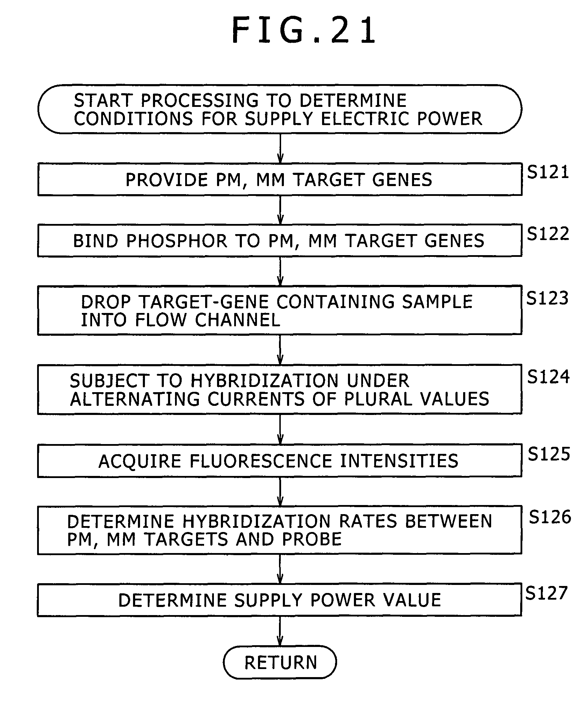

In step S95, supply power condition determination processing to be described subsequently herein with reference to FIG. 21 is performed, and the processing is then ended.

By the processing as described above, the DNA chip 43 to be employed in the processing in the course of the experiment is formed, the fluorescence intensity-hybridization abundance conversion equation required for estimating an expression abundance on the basis of a fluorescence intensity and the probe-target binding strength matrix can be obtained, and the supply power conditions to be produced at the AC supply unit 41 of the hybridization unit 22 and to be supplied to the electromagnetic induction generator unit 42 are determined.

With reference to a flow chart of FIG. 21, a description will next be made about the supply power condition determination processing to be performed in step S95 of FIG. 20. The supply power condition determination processing is performed by using the preparation unit 21, hybridization unit 22, acquisition unit 23 and expression abundance estimation unit 24 or a system capable of performing processing equivalent to them

In step S121, the PM target gene 142 and MM target gene 143 are provided.

Described specifically, the PM target gene 142 is an mRNA as a measurement target, and the MM target 143 is an mRNA the hybridization probability of which with the probe 141 prepared to permit hybridization while assuming the PM target gene 142 is expected to give a largest expression except for the PM target gene 142. Here, it is suited to use such a pair of PM target gene 142 and MM target gene 143 as bringing about highest hybridization probability (binding strength) between the PM target gene 142 and MM target gene 143.

In step S122, the preparation unit 121 binds a phosphor such as, for example, cy3 or cy5, for example, to the PM target gene 142 and MM target gene 143.

In step S123, the preparation unit 121 drops a solution, which contains the PM target gene and MM target gene, into the flow channel of the expression-analyzing reaction channel 81 of the formed DNA chip 43 in a similar manner as that explained with reference to step S61 and stepS62 of FIG. 19. The DNA chip 43 is then mounted in the hybridization unit 22.

In step S124, the hybridization unit 22 generates alternating currents of plural values at the AC supply unit 41 and supplies them to the electromagnetic induction generator unit 42. As described with reference to FIG. 13 and FIG. 14, the PM target gene 142 and MM target gene 143 in the flow channel of the expression-analyzing reaction channel 81 of the DNA chip 43 are caused to electrophoretically migrate by electromagnetic induction. In this manner, DNA chips 43 subjected to hybridization at respective AC voltage values are obtained. The hybridization unit 22 removes the probes not immobilized by the hybridization, that is, the single-stranded probe and target from the respective DNA chips 43, for example, by washing.

In step S125, the acquisition unit 23 acquires fluorescence intensities from the DNA chips 43 subjected to the hybridization at the respective AC voltage values in a similar manner as in the case described with reference to step S13 of FIG. 18.

In step S126, the expression abundance estimation unit 24, specifically the hybridization rate estimation unit 424, in a similar manner as in the case explained with reference to step S14 of FIG. 18, estimates expression abundances, determines the hybridization rates between the PPM target gene 142 and MM target gene 143 and the probe 141, and to the supply power value determination unit 431, supplies the hybridization rates of the PM target gene 142 and MM target gene 143 in correspondence to the respective AC voltage values applied, in other words, the respective energy quantities applied for electrophoretic migration.

In step S127, the supply power value determination unit 28, specifically the supply power value determination unit 431, as described, for example, with reference to FIG. 9 or FIG. 10 or with reference to the formula (1) through formula (6), determines the supply power value of an AC voltage generated by the AC supply unit 41 and most suited for being supplied to the electromagnetic induction generator unit 42 in the hybridization performed in the processing in the course of the experiment as described with reference to FIG. 18, specifically a power value capable of applying the electrophoretically-migrating target genes energy required to dissociate the MM target gene 143 non-specifically hybridized with the probe 141 without dissociating the PM target gene 142 correctly hybridized with the probe 141, and supplies the power value to the AC supply unit 41 of the hybridization unit 22, and then, the processing is ended.

By the processing as described above, the conditions for the supply electric power to be generated at the AC supply unit 42 of the hybridization unit 22 and to be supplied to the electromagnetic induction generator unit 42 are determined to effectively perform hybridization.

As described above, the present invention has made it possible to increase the frequency of binding between the target gene and the probe by designing the flow channel, in which a target-containing solution is dropped, into a closed form (which can be any form such as a circular form, polygonal form or the like). The state of hybridization after an elapse of sufficient time since the initiation of a supply of an alternating current from the AC supply unit 41 to the magnetic field generating electroconductor 111 of the electromagnetic induction generator unit 42 becomes very good.

Further, it is possible to determine energy required to dissociate the MM target gene 143 non-specifically hybridized with the probe 141 without dissociating the PM target gene 142 correctly hybridized with the probe 141, and also to determine supply electric energy required to apply such energy to the electrophoretically-migrating target genes. Owing to the application of such energy to the target genes upon hybridization, the non-specific hybridization can be dissociated without dissociating the specific hybridization so that the detection accuracy of the target gene is improved.

Use of a circuit that permits an electric current only in a constant direction such as, for example, diodes or the like makes it possible to cancel electric fields, which are produced to negate a magnetic field generated by an AC current, in only one direction and to inhibit cancellation of the electric fields in the other direction (to maintain the electric fields in the other direction). This enables to apply an electric field of predetermined strength (strength corresponding to the energy sufficient to dissociate non-specific hybridization without dissociating specific hybridization) in a constant direction to a flow channel of a closed configuration.

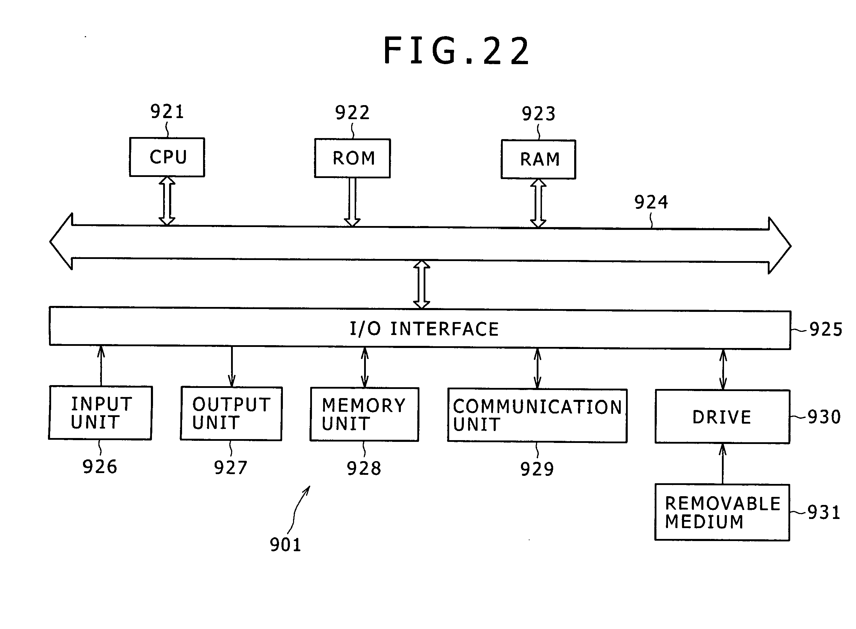

The above-mentioned series of processing can be performed by hardware or can be performed by software. In this case, the biological information processing sub-system 1 can be constructed, for example, by a personal computer 901 shown in FIG. 22.

In FIG. 22, CPU (central processing unit) 921 performs various processing in accordance with a program stored in ROM (Read Only Memory) 922 or a program loaded from a memory unit 928 to RAM (Random Access Memory) 923. In RAM 923, data which CPU 921 requires in performing various processing and like data are also stored as needed.

CPU 921, ROM 922 and RAM 923 are mutually connected via a bus 924. To this bus 924, an input/output interface 925 is also connected.

Connected to the input/output interface 925 are an input unit 926 composed of a keyboard, mouse and the like, a display composed of CRT (Cathode Ray Tube), LCD (Liquid Crystal Display) or the like, an output unit 927 composed of a speaker or the like, a memory unit 928 composed of a hard disk or the like, and a communication unit 929 composed of a modem or the like. The communication unit 929 performs communication processing via networks including internet.

To the input/output interface 925, a drive 930 is also connected as needed. A removable medium 931 such as a magnetic disk, optical disk, magnetooptic disk or semiconductor memory is mounted there as desired, and a computer program read from the removable medium can be installed in the memory unit 928 as needed.

To perform the series of processing by software, on the other hand, a computer with a program, which constitutes the software, incorporated in dedicated hardware can be used, or such a program can be installed from a network or recording medium, for example, in a general-purpose computer which can perform diverse functions by installing various programs.

As depicted in FIG. 22, this recording medium may comprise the removable medium 931 composed of a magnetic disk (including a floppy disk), optical disk (CD-ROM (Compact Disk-Read Only Memory), DVD (including (Digital Versatile Disk)) or magnetooptic disk (including MD (Mini-Disk) distributed in addition to a computer main body to provide the user with the program, or as an alternative, a hard disk or the like furnished to the user in a state assembled beforehand in a computer main body and included in ROM 922 or memory unit 928 in which the program is recorded.

It is to be noted that in this specification, steps which describe the program to be recorded in a recording medium include processing executed in parallel or individually instead of being necessarily performed in a chronological order, to say nothing of processing conducted in a chronological order in the described order.

It is also to be noted that in this specification, the term "system" means a logical assembly of plural units (or function modules for realizing specific functions) irrespective of whether or not the units or function modules exist in a single housing.

**Claims**

1. A bioreaction execution system for subjecting a first biological substance, which is immobilized in a reaction region arranged in at least one closed flow channel of a substrate with said flow channel formed thereon, and a second biological substance, which is bioreactive with the first biological substance, to a bioreaction, comprising:

    an electromagnetic induction generator unit for generating electromagnetic induction to produce an electric field of a predetermined direction along said flow channel such that the second biological substance contained in a solution dropped into said flow channel of said substrate mounted on said system is caused to electrophoretically migrate.

2. The bioreaction execution system according to claim 1, wherein said flow channel formed on said substrate is circular.

3. The bioreaction execution system according to claim 1, wherein:

    the second biological substance comprises a third biological substance specifically bioreactive with the first biological substance and a fourth substance non-specifically bioreactive with the first biological substance; and said electromagnetic induction generator unit generates electromagnetic induction such that energy to be applied by the electric field of the predetermined direction to cause the electrophoretic migration of the second biological substance in said flow channel can dissociate an association formed by a bioreaction between the first biological substance and the fourth biological substance without dissociating an association formed by a bioreaction between the first biological substance and the third biological substance.

4. The bioreaction execution system according to claim 3, further comprising:

    an AC supply unit for supplying an AC voltage to said electromagnetic induction generator unit,

    wherein the AC voltage to be supplied by said AC supply unit to said electromagnetic induction generator unit has electric energy sufficient to generate electromagnetic induction such that the energy to be applied by the electric field of the predetermined direction to cause the electrophoretic migration of the second biological substance in said flow channel cannot dissociate the association formed by the bioreaction between the first biological substance and the third biological substance but can dissociate the association formed by the bioreaction between the first biological substance and the fourth biological substance.

**5.** The bioreaction execution system according to claim 1, wherein said electromagnetic induction generator unit is provided with:

> a coil-shaped electroconductor for receiving a supply of an alternating current and allowing a current to flow alternately in two directions depending on a polarity of said alternating current; and
> an electric field canceller for canceling production of one of electric fields, which are produced in two directions to negate a magnetic field generated by said current flowing through said electroconductor, by permitting only a current generated by the one electric field and not permitting a current generated by the other electric field.

**6.** The bioreaction execution system according to claim 5, wherein:

> said flow channel formed on said substrate is circular; and
> said electroconductor and said electric field canceller are arranged above and below said circular flow channel, respectively, with said circular flow channel located therein.

**7.** The bioreaction execution system according to claim 5, wherein:

> said substrate is provided with plural flow channels, which are as defined in claim 5, in a concentric pattern;
> said electroconductors and electric field cancellers, which are as defined in claim 5, are arranged above and below said flow channels, respectively, with the corresponding flow channels located therein;
> said electroconductors each conducts an AC current opposite in polarity to those conducted through adjacent one(s) of said electroconductors; and
> said electric field cancellers cancel electric fields of a same direction.

**8.** A method for executing a bioreaction in a bioreaction execution system that subjects a first biological substance, which is immobilized in a reaction region arranged in at least one closed flow channel formed on a substrate to permit flowing of a solution dropped onto said substrate, and a second biological substance, which is bioreactive with the first biological substance, to said bioreaction, comprising the step of:

> generating electromagnetic induction to produce an electric field of a predetermined direction along said flow channel such that the second biological substance contained in said dropped into said flow channel of said substrate is caused to electrophoretically migrate.

**9.** A DNA chip comprising:

> a substrate,
> a flow channel formed on said substrate and including a concave channel of a closed form,
> a reaction region arranged in said flow channel to immobilize a first biological substance therein such that the first biological substance is allowed to undergo a bioreaction with a second biological substance contained as a detection target in a solution dropped into said flow channel.

**10.** An information processing system for executing processing, which determines an energy quantity to be applied for electrophoretic migration of a second biological substance bioreactive with a first biological substance and contained in a solution dropped into at least one closed flow channel formed on a substrate, in a bioreaction execution system for subjecting the first biological substance, which is immobilized in a reaction region arranged in said flow channel, and the second biological substance to a bioreaction, comprising:

> an acquisition means for acquiring, on said substrate with a solution containing a third biological substance specifically bioreactive with the first biological substance and a fourth substance non-specifically bioreactive with the first biological substance dropped as the second biological substance into said flow channel, parameters corresponding to bioreaction rates of the third biological substance and fourth biological substance in states that different energy quantities have been applied, respectively,

a calculation means for calculating, based on the parameters acquired by said acquisition means, the bioreaction rates of the third biological substance and fourth biological substance in the states that the different energy quantities have been applied, and

an energy determination means for determining, based on the bioreaction rates calculated by said calculation means, an energy quantity that can dissociate an association formed by a bioreaction between the first biological substance and the fourth biological substance without dissociating an association formed by a bioreaction between the first biological substance and the third biological substance.

11. An information processing method for an information processing system that executes processing, which determines an energy quantity to be applied for electrophoretic migration of a second biological substance bioreactive with a first biological substance and contained in a solution dropped into at least one closed flow channel formed on a substrate, in a bioreaction execution system for subjecting the first biological substance, which is immobilized in a reaction region arranged in said flow channel, and the second biological substance to a bioreaction, comprising:

an acquisition step for acquiring, on said substrate with a solution containing a third biological substance specifically bioreactive with the first biological substance and a fourth substance non-specifically bioreactive with the first biological substance dropped as the second biological substance into said flow channel, parameters corresponding to bioreaction rates of the third biological substance and fourth biological substance in states that different energy quantities have been applied, respectively,

a calculation step for calculating, based on the parameters acquired by processing in said acquisition step, the bioreaction rates of the third biological substance and fourth biological substance in the states that the different energy quantities have been applied, and

an energy determination step for determining, based on the bioreaction rates calculated by processing in said calculation step, an energy quantity that can dissociate an association formed by a bioreaction between the first biological substance and the fourth biological substance without dissociating an association formed by a bioreaction between the first biological substance and the third biological substance.

12. A program for making a computer execute processing, which determines an energy quantity to be applied for electrophoretic migration of a second biological substance bioreactive with a first biological substance and contained in a solution dropped into at least one closed flow channel formed on a substrate, in a bioreaction execution system for subjecting the first biological substance, which is immobilized in a reaction region arranged in said flow channel, and the second biological substance to a bioreaction, comprising:

an acquisition control step for controlling, on said substrate with a solution containing a third biological substance specifically bioreactive with the first biological substance and a fourth substance non-specifically bioreactive with the first biological substance dropped as the second biological substance into said flow channel, acquisition of parameters corresponding to bioreaction rates of the third biological substance and fourth biological substance in states that different energy quantities have been applied, respectively,

a calculation step for calculating, based on the parameters acquisition of which was controlled by processing in said acquisition step, the bioreaction rates of the third biological substance and fourth biological substance in the states that the different energy quantities have been applied, and

an energy determination step for determining, based on the bioreaction rates calculated by processing in said calculation step, an energy quantity that can dissociate an association formed by a bioreaction between the first biological substance and the fourth biological substance without dissociating an association formed by a bioreaction between the first biological substance and the third biological substance.

13. A recording medium with a program according to claim 12 recorded thereon.

# FIG.1

# FIG.2

41

42

AC SUPPLY UNIT

ELECTROMAGNETIC
INDUCTION
GENERATOR UNIT

DNA CHIP ～43

22

ELECTROMAGNETIC
INDUCTION COIL

| | |
|---|---|
| 68 POWER METER (SWR METER) | |
| 65 ELECTRIC POWER SETTING UNIT | 64 E-CLASS AMPLIFIER | 67 VARIABLE POWER SUPPLY 0 - 120V |
| | 63 RF POWER AMPLIFIER | 66 AC100V SUPPLY UNIT |
| | 62 OSCILLATOR | |
| | 61 FREQUENCY SETTING UNIT | |

41

FIG. 3

# F I G . 4

83

81-1

81-2

43

# FIG.5

# FIG.6

# FIG.7

121

122

113

# FIG.8

# FIG.9

# FIG.10

HYBRIDIZATION RATE

MAXIMUM VALUE

181

50%

182

MINIMUM VALUE

ELECTRIC POWER

SUPPLY POWER VALUE

EP 1 865 322 A1

EP 1 865 322 A1

**FIG.11A**

CURRENT

CLOCKWISE

COUNTERCLOCKWISE

TIME

**FIG.11B**

FIG.12A

FIG.12B

# FIG.13

# FIG.14

# FIG.15

EP 1 865 322 A1

# FIG.16A

# FIG.16B

EP 1 865 322 A1

# FIG.17

EP 1 865 322 A1

# FIG.18

```
        ┌─────────────────────────────┐
        │  START PROCESSING IN THE    │
        │  COURSE OF EXPERIMENT       │
        └─────────────────────────────┘
                      │
                      ▼
        ┌─────────────────────────────┐
        ║     PREPARE TARGETS         ║  S11
        └─────────────────────────────┘
                      │
                      ▼
        ┌─────────────────────────────┐
        ║       HYBRIDIZATION         ║  S12
        └─────────────────────────────┘
                      │
                      ▼
        ┌─────────────────────────────┐
        │ ACQUIRE FLUORESCENCE INTENSITY │  S13
        └─────────────────────────────┘
                      │
                      ▼
        ┌─────────────────────────────┐
        │ ESTIMATE EXPRESSION ABUNDANCE │  S14
        └─────────────────────────────┘
                      │
                      ▼
        ┌─────────────────────────────┐
        │      STANDARDIZE DATA       │  S15
        └─────────────────────────────┘
                      │
                      ▼
        ┌─────────────────────────────┐
        │ OUTPUT EXPRESSION PROFILE DATA │  S16
        └─────────────────────────────┘
                      │
                      ▼
                ┌───────────┐
                │  RETURN   │
                └───────────┘
```

# FIG.19

START HYBRIDIZATION PROCESSING

ADD CONTROL TARGET TO
TARGET-CONTAINING SOLUTION — S61

DROP TARGET-CONTAINING SOLUTION
INTO FLOW CHANNEL — S62

SUBJECT TO ELECTROPHORETIC MIGRATION
BY ELECTROMAGNETIC INDUCTION — S63

REMOVE UNHYBRIDIZED SINGLE-STRANDED
PROBE AND TARGETS — S64

INTRODUCE INTERCALATOR — S65

RETURN

# FIG.20

```
START PROCESSING IN PRELIMINARY WORK
```

| | |
|---|---|
| DESIGN PROBE | S91 |

| | |
|---|---|
| PREPARE DNA CHIP | S92 |

| | |
|---|---|
| ACQUIRE FLUORESCENCE INTENSITY-HYBRIDIZATION ABUNDANCE CONVERSION EQUATION | S93 |

| | |
|---|---|
| ACQUIRE PROBE-TARGET BINDING STRENGTH MATRIX | S94 |

| | |
|---|---|
| PERFORM PROCESSING TO DETERMINE CONDITIONS FOR SUPPLY ELECTRIC POWER | S95 |

```
END
```

# F I G . 2 1

START PROCESSING TO DETERMINE
CONDITIONS FOR SUPPLY ELECTRIC POWER

PROVIDE PM, MM TARGET GENES — S121

BIND PHOSPHOR TO PM, MM TARGET GENES — S122

DROP TARGET-GENE CONTAINING SAMPLE
INTO FLOW CHANNEL — S123

SUBJECT TO HYBRIDIZATION UNDER
ALTERNATING CURRENTS OF PLURAL VALUES — S124

ACQUIRE FLUORESCENCE INTENSITIES — S125

DETERMINE HYBRIDIZATION RATES BETWEEN
PM, MM TARGETS AND PROBE — S126

DETERMINE SUPPLY POWER VALUE — S127

RETURN

# FIG.22

## INTERNATIONAL SEARCH REPORT

| | International application No. |
|---|---|
| | PCT/JP2006/306486 |

**A. CLASSIFICATION OF SUBJECT MATTER**
*G01N33/53*(2006.01)*,C12M1/00*(2006.01)*,C12N15/09*(2006.01)*,G01N27/447*(2006.01)*,*
*G01N37/00*(2006.01)

According to International Patent Classification (IPC) or to both national classification and IPC

**B. FIELDS SEARCHED**

Minimum documentation searched (classification system followed by classification symbols)
*G01N33/53*(2006.01)*,C12M1/00*(2006.01)*,C12N15/09*(2006.01)*,G01N27/447*(2006.01)*,*
*G01N37/00*(2006.01)

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | SOSNOWSKI, R.G. et al., "Rapid determination of single base mismatch mutations in DNA hybrids by direct electric field control", Proc.Natl.Acad.Sci.USA, 1997, Vol.94, pages 1119 to 1123, Abstract, Fig. 1 | 1,2,8,9 |
| Y | JP 2002-333444 A (Matsushita Electric Industrial Co., Ltd.), 22 November, 2002 (22.11.02), Claims (Family: none) | 1,2,8,9 |
| Y | JP 2002-501174 A (Remacle Jose), 15 January, 2002 (15.01.02), Claims & WO 1999/035499 A1   & US 2002/0177144 A1 & EP 1044375 A | 2 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 June, 2006 (15.06.06) | 27 June, 2006 (27.06.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

EP 1 865 322 A1

**INTERNATIONAL SEARCH REPORT**

International application No.

PCT/JP2006/306486

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2003-156476 A  (Matsushita Electric Industrial Co., Ltd.), 30 May, 2003 (30.05.03), (Family: none) | 1-13 |
| A | JP 2004-045376 A  (Sony Corp.), 12 February, 2004 (12.02.04), & WO 2003/098216 A1    & US 2005/0069880 A & EP 1507146 A1 | 1-13 |
| A | JP 2003-185665 A  (Samsung Electronics Co., Ltd.), 03 July, 2003 (03.07.03), & US 2003/0077649 A1    & EP 1306449 A2 | 1-13 |

Form PCT/ISA/210 (continuation of second sheet) (April 2005)

**EP 1 865 322 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002071688 A
- JP 2002267668 A
- JP 2003028862 A